# EUROPEAN PATENT APPLICATION

(11) **EP 4 253 379 A2**
(43) Date of publication of application: **04.10.2023**
(21) Application number: 23168252.7
(22) Date of filing: 16.10.2020
(51) Int. Cl.: C07D 405/04

(54) **METHODS FOR THE PREPARATION OF 5-BROMO-2-(3-CHLORO-PYRIDIN-2-YL)-2H-PYRAZOLE-3-CARBOXYLIC ACID**

(30) Priority: 18.10.2019 US 201962916827 P
(62) Divisional of application: 20804066.7
(71) Applicant: FMC Corporation, Philadelphia, Pennsylvania 19104 (US); FMC Agro Singapore Pte. Ltd., Singapore 018983 (SG)
(72) Inventor: CHEN, Yuzhong, Philadelphia, PA 19104 (US); FREUDENBERGER, John Herbert, Philadelphia, PA 19104 (US); WRIGHT, James, Philadelphia, PA 19104 (US)
(74) Representative: Dehns

(57) **Abstract**

The invention is directed to methods for preparing intermediates which are useful for making insecticidal anthranilamide compounds such as chlorantraniliprole and cyantraniliprole.

## Description

### CROSS-REFERENCE TO RELATED APPLICATION

This application claims the benefit of U.S. Provisional Application No. 62/916,827 filed October 18, 2019.

### FIELD OF INVENTION

This disclosure is directed to novel methods of synthesizing 5-Bromo-2-(3-chloro-pyridin-2-yl)-2H-pyrazole-3-carboxylic acid. Compounds prepared by the methods disclosed herein are useful for preparation of certain anthranilamide compounds that are of interest as insecticides, such as, for example, the insecticides chlorantraniliprole and cyantraniliprole.

### BACKGROUND

The present disclosure provides novel methods useful for preparing 5-Bromo-2-(3-chloro-pyridin-2-yl)-2H-pyrazole-3-carboxylic acid and derivatives thereof. The benefits of the methods of the present disclosure compared to previous methods are numerous and include improved overall yield, reduced cost, reduced waste, simplified operation complexity, and fewer steps in a linear sequence (i.e. more highly convergent synthesis).

The disclosed methods provide an overall yield of about 50% with commercially available and easily handled reagents.

### BRIEF DESCRIPTION

In one aspect, provided herein is a method of preparing a compound of Formula VI, wherein 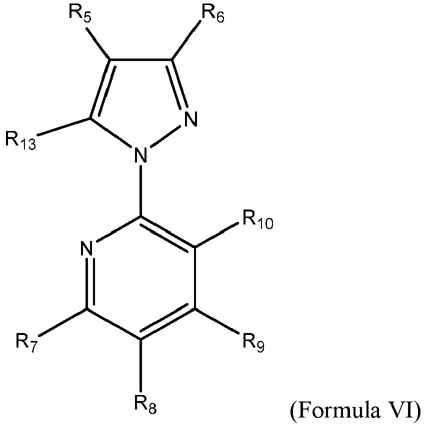
each of R₅ - R₁₀ is independently selected from hydrogen and halogen; and
R₁₃ is an organic acid, the method comprising
   I) forming a mixture comprising
      A) a compound of Formula V, wherein 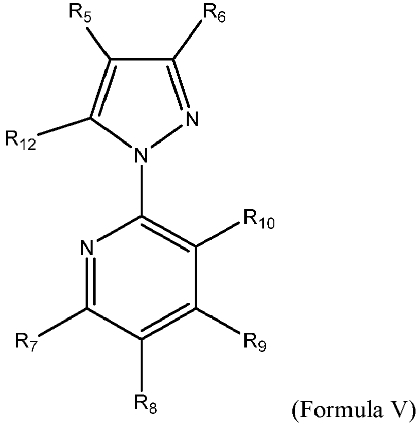
         each of R₅ - R₁₀ is independently selected from hydrogen and halogen;
         R₁₂ is selected from ether, ester, and nitrile; and wherein the compound of Formula V is prepared according to a method comprising
            i) forming a mixture comprising
               a) a compound of Formula III, wherein 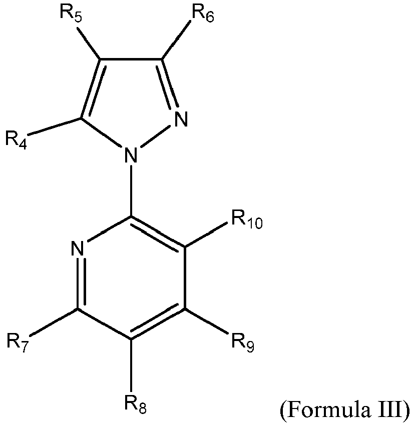
                  R₄ is hydrogen;
                  each of R₅ - R₁₀ is independently selected from hydrogen and halogen; and
                  wherein the compound of Formula III is prepared according to a method comprising
                     IA) forming a mixture comprising
                        AA) a compound of Formula II, wherein 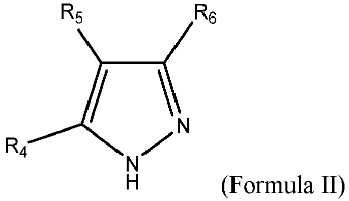
                           each of R₄, R₅, and R₆ is independently selected from hydrogen and halogen;
                           wherein at least one of R₄, R₅, and R₆ is hydrogen; and
                           wherein the compound of Formula II is prepared according to a method comprising
                              ia) forming a mixture comprising
                                 aa) a compound of Formula VIII, wherein 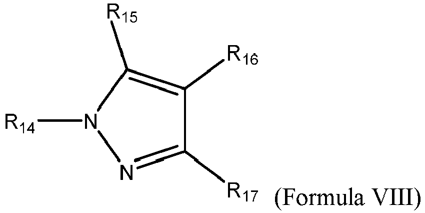
                                    R₁₄ is selected from substituted or unsubstituted carbocycle and substituted or unsubstituted heterocycle;
                                    each of R₁₅, R₁₆, and R₁₇ is independently selected from hydrogen and halogen; and
                                    wherein at least one of R₁₅, R₁₆, and R₁₇ is a halogen;
                                 bb) a solvent; and
                                 cc) an acid; and
                              iia) reacting the mixture;
                        BB) a compound of Formula IV, wherein 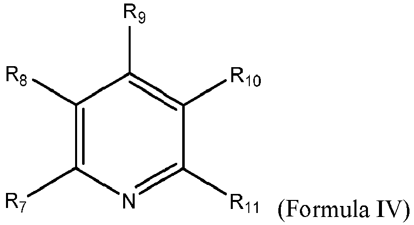 each of R₇ - R₁₁ is independently selected from hydrogen and halogen;
                        CC) a solvent;
                        DD) an inorganic base; and
                        EE) optionally an additive; and
                     IIA) reacting the mixture;
               b) a solvent;
               c) a base comprising
                  a compound comprising a metal; and
                  optionally a first amine base;
               d) optionally an additive; and
               e) optionally a second amine base; and
            ii) reacting the mixture with an organic compound; and
      B) a metal hydroxide; and
   II) reacting the mixture.

In one aspect, provided herein is a method of preparing a compound of Formula VI, wherein 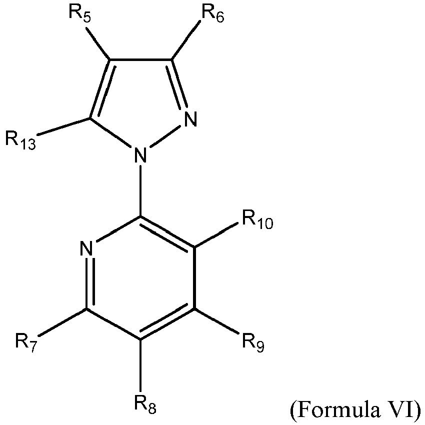
each of R₅ - R₁₀ is independently selected from hydrogen and halogen; and
R₁₃ is an organic acid, the method comprising
   I) forming a mixture comprising
      A) a compound of Formula III, wherein 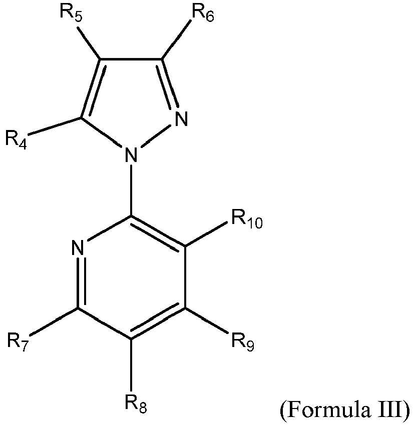
         R₄ is hydrogen;
         each of R₅ - R₁₀ is independently selected from hydrogen and halogen; and
         wherein the compound of Formula III is prepared according to a method comprising
            i) forming a mixture comprising
               a) a compound of Formula II, wherein 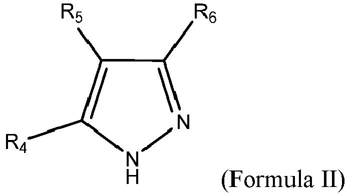
                  each of R₄, R₅, and R₆ is independently selected from hydrogen and halogen;
                  wherein at least one of R₄, R₅, and R₆ is hydrogen; and
                  wherein the compound of Formula II is prepared according to a method comprising
                     IA) forming a mixture comprising
                        AA) a compound of Formula VIII, wherein 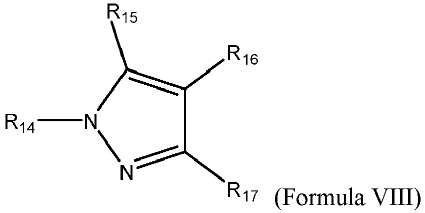
                           R₁₄ is selected from substituted or unsubstituted carbocycle and substituted or unsubstituted heterocycle;
                           each of R₁₅, R₁₆, and R₁₇ is independently selected from hydrogen and halogen; and
                           wherein at least one of R₁₅, R₁₆, and R₁₇ is a halogen;
                        BB) a solvent; and
                        CC) an acid; and
                     IIa) reacting the mixture;
               b) a compound of Formula IV, wherein 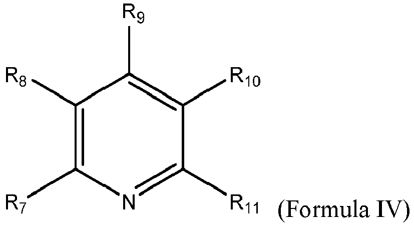 each of R₇ - R₁₁ is independently selected from hydrogen and halogen;
               c) a solvent;
               d) an inorganic base; and
               e) optionally an additive; and
            ii) reacting the mixture;
      B) a solvent;
      C) a base comprising
         a compound comprising a metal; and
         optionally a first amine base;
      D) optionally an additive; and
      E) optionally a second amine base; and
   II) reacting the mixture with a carbonyl-containing compound.

In one aspect, provided herein is a method of preparing a compound of Formula II, wherein 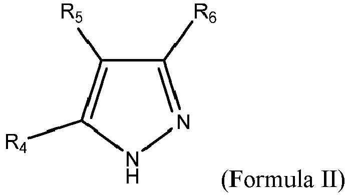
each of R₄, R₅, and R₆ is independently selected from hydrogen and halogen; and
wherein at least one of R₄, R₅, and R₆ is hydrogen, the method comprising
   I) forming a mixture comprising
      A) a compound of Formula VIII, wherein 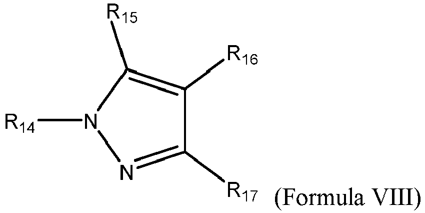
         R₁₄ is selected from substituted or unsubstituted carbocycle and substituted or unsubstituted heterocycle;
         each of R₁₅, R₁₆, and R₁₇ is independently selected from hydrogen and halogen; and
         wherein at least one of R₁₅, R₁₆, and R₁₇ is a halogen;
      B) a solvent; and
      C) an acid; and
   II) reacting the mixture.

In one aspect, provided herein is a method of preparing a compound of Formula VII, wherein 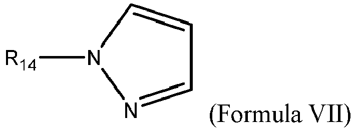 R₁₄ is selected from substituted or unsubstituted carbocycle and substituted or unsubstituted heterocycle, the method comprising
I) forming a mixture comprising
   A) pyrazole or a pyrazole derivative;
   B) a cycloalkene or heterocycloalkene;
   C) optionally a solvent; and
   D) an acid; and
II) reacting the mixture.

In one aspect, provided herein is a method of preparing a compound of Formula VIII, wherein 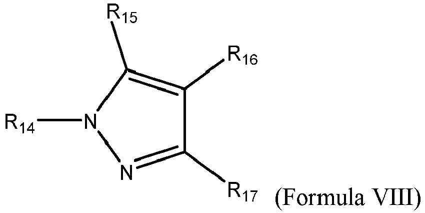
R₁₄ is selected from substituted or unsubstituted carbocycle and substituted or unsubstituted heterocycle;
each of R₁₅, R₁₆, and R₁₇ is independently selected from hydrogen and halogen; and
wherein at least one of R₁₅, R₁₆, and R₁₇ is a halogen, the method comprising
   I) forming a mixture comprising
      A) the compound of Formula VII, wherein 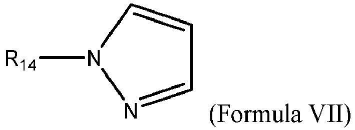 R₁₄ is selected from substituted or unsubstituted carbocycle and substituted or unsubstituted heterocycle;
      B) a solvent;
      C) a base comprising
         a compound comprising a metal; and
         optionally a first amine base; and
      D) optionally a second amine base; and
   II) reacting the mixture with a halogenation reagent.

In one aspect, provided herein is a method of preparing a compound of Formula V, wherein 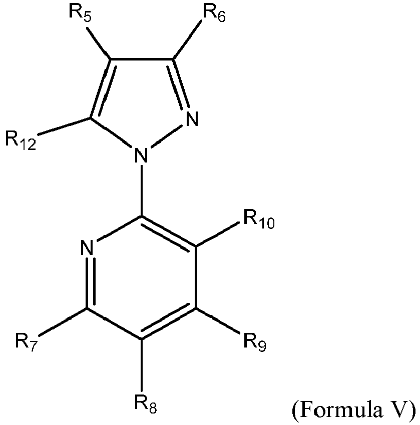
each of R₅ - R₁₀ is independently selected from hydrogen and halogen; and
R₁₂ is selected from ether, ester, and nitrile, the method comprising
   i) forming a mixture comprising
      a) a compound of Formula III, wherein 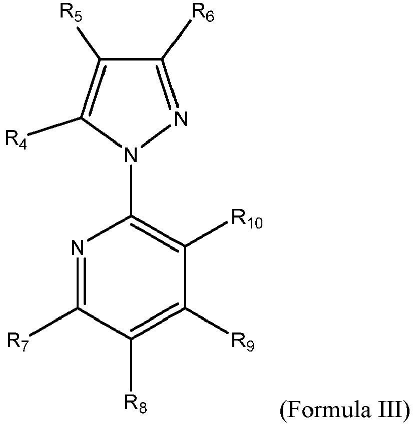
         R₄ is hydrogen;
         each of R₅ - R₁₀ is independently selected from hydrogen and halogen; and
         wherein the compound of Formula III is prepared according to a method comprising
            IA) forming a mixture comprising
               AA) a compound of Formula II, wherein 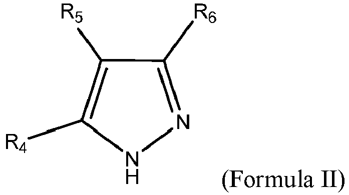
                  each of R₄, R₅, and R₆ is independently selected from hydrogen and halogen;
                  wherein at least one of R₄, R₅, and R₆ is hydrogen; and
                  wherein the compound of Formula II is prepared according to a method comprising
                     ia) forming a mixture comprising
                        aa) a compound of Formula VIII, wherein 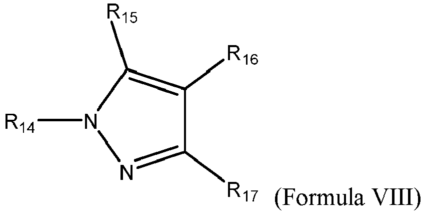
                           R₁₄ is selected from substituted or unsubstituted carbocycle and substituted or unsubstituted heterocycle;
                           each of R₁₅, R₁₆, and R₁₇ is independently selected from hydrogen and halogen; and
                           wherein at least one of R₁₅, R₁₆, and R₁₇ is a halogen;
                        bb) a solvent; and
                        cc) an acid; and
                     iia) reacting the mixture;
               BB) a compound of Formula IV, wherein 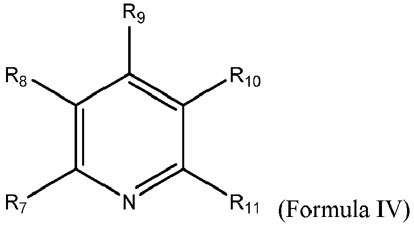 each of R₇ - R₁₁ is independently selected from hydrogen and halogen;
               CC) a solvent;
               DD) an inorganic base; and
               EE) optionally an additive; and
            IIA) reacting the mixture;
      b) a solvent;
      c) a base comprising
         a compound comprising a metal; and
         optionally a first amine base;
      d) optionally an additive; and
      e) optionally a second amine base; and
   ii) reacting the mixture with an organic compound.

In one aspect, provided herein is a method of preparing a compound of Formula III, wherein 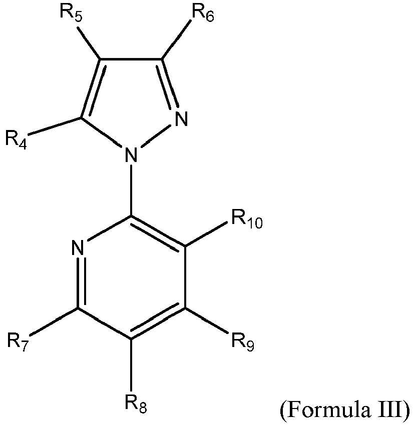
R₄ is hydrogen; and
each of R₅ - R₁₀ is independently selected from hydrogen and halogen, the method comprising
   i) forming a mixture comprising
      a) a compound of Formula II, wherein 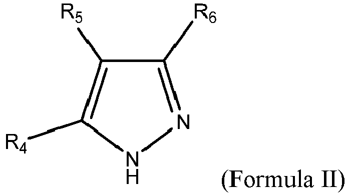
         each of R₄, R₅, and R₆ is independently selected from hydrogen and halogen;
         wherein at least one of R₄, R₅, and R₆ is hydrogen; and
         wherein the compound of Formula II is prepared according to a method comprising
            IA) forming a mixture comprising
               AA) a compound of Formula VIII, wherein 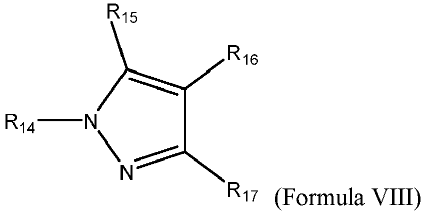
                  R₁₄ is selected from substituted or unsubstituted carbocycle and substituted or unsubstituted heterocycle;
                  each of R₁₅, R₁₆, and R₁₇ is independently selected from hydrogen and halogen; and
                  wherein at least one of R₁₅, R₁₆, and R₁₇ is a halogen;
               BB) a solvent; and
               CC) an acid; and
            IIa) reacting the mixture;
      b) a compound of Formula IV, wherein 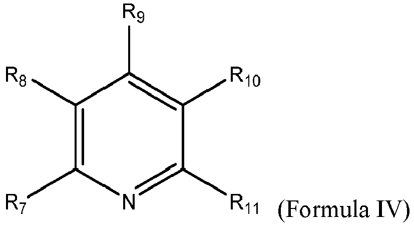 each of R₇ - R₁₁ is independently selected from hydrogen and halogen;
      c) a solvent;
      d) an inorganic base; and
      e) optionally an additive; and
   ii) reacting the mixture.

### DETAILED DESCRIPTION OF THE DISCLOSURE

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process or method.

The transitional phrase "consisting of' excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of' is used to define a composition or method that includes materials, steps, features, components, or elements, in addition to those literally disclosed, provided that these additional materials, steps, features, components, or elements do not materially affect the basic and novel characteristic(s) of the claimed invention. The term "consisting essentially of' occupies a middle ground between "comprising" and "consisting of".

Where an invention or a portion thereof is defined with an openended term such as "comprising," it should be readily understood that (unless otherwise stated) the description should be interpreted to also describe such an invention using the terms "consisting essentially of" or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As used herein, the term "about" means plus or minus 10% of the value.

The term "halogen", either alone or in compound words such as "haloalkyl", includes fluorine, chlorine, bromine or iodine. Further, when used in compound words such as "haloalkyl", said alkyl may be partially or fully substituted with halogen atoms which may be the same or different.

When a group contains a substituent which can be hydrogen, for example R⁴, then, when this substituent is taken as hydrogen, it is recognized that this is equivalent to said group being unsubstituted.

The term "organic base" includes, without limitation, amine compounds (e.g., primary, secondary and tertiary amines), heterocycles including nitrogen-containing heterocycles, and ammonium hydroxide.

The term "inorganic base" includes, without limitation, inorganic compounds with the ability to react with, or neutralize, acids to form salts, such as, for example, metal salts of hydroxide, carbonate, bicarbonate and phosphate.

The term "halogenation reagent" includes, without limitation, halogens and inorganic compounds, such as, for example, bromine, N-bromosuccinimide, 1,3-dibromo-5,5-dimethylhylhydantoin, 1,2-dibromotetrachloroethane, tetrabutylammonium tribromide, quaternary ammonium salts of tribromide, 1,2-dibromotrichloroethane, diethyl dibromomalonate, and combinations thereof.

The term "phase transfer catalyst" includes compounds that facilitate the migration of a reactant from one phase into another phase where a reaction occurs. Phase transfer catalysis refers to the acceleration of the reaction upon the addition of the phase transfer catalyst.

The term "ester" includes, without limitation, a functional group comprising an ester bond (C(=O)-O-). In some aspects, the functional group comprising an ester bond is an alkyl (or cycloalkyl) having one to eight carbon atoms, like methyl, ethyl, 1 -propyl, 2-propyl, 1 - butyl, 1-methylheptyl (meptyl), etc.

The term "ether" includes, without limitation, a functional group comprising an ether bond (C-O-C).

The term "nitrile" includes, without limitation, a functional group comprising a nitrile bond (-C≡N).

The term "carboxylic acid" includes, without limitation, a functional group comprising a carboxylic acid bond (C(=O)-OH).

The term "organic acid" includes, without limitation, a functional group that confers acidity and consists of atoms selected from carbon, nitrogen, oxygen, and hydrogen.

The term "carbocycle" includes, without limitation, a functional group comprising a ring or ring system wherein the atoms forming the ring backbone are selected only from carbon.

The term "heterocycle" includes, without limitation, a functional group comprising a ring or ring system in which at least one atom forming the ring backbone is not carbon, e.g., nitrogen, oxygen, or sulfur.

The term "saturated" refers to a functional group comprising a backbone consisting of atoms linked to one another by single bonds; unless otherwise specified, the remaining atom valences are occupied by hydrogen atoms.

The term "unsaturated" refers to a functional group comprising a backbone comprising at least one double bond. Unless otherwise stated, an "unsaturated" functional group may be partially unsaturated or fully unsaturated.

The term "cycloalkene" includes, without limitation, a functional group comprising a ring or ring system wherein the atoms forming the ring backbone are selected only from carbon, and wherein the ring or ring system comprises at least one double bond.

The term "heterocycloalkene" includes, without limitation, a functional group comprising a ring or ring system in which at least one atom forming the ring backbone is not carbon, e.g., nitrogen, oxygen, or sulfur, and wherein the ring or ring system comprises at least one double bond.

Certain compounds of this invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers.

The embodiments of this disclosure include:
Embodiment 1. A method of preparing a compound of Formula VI, wherein 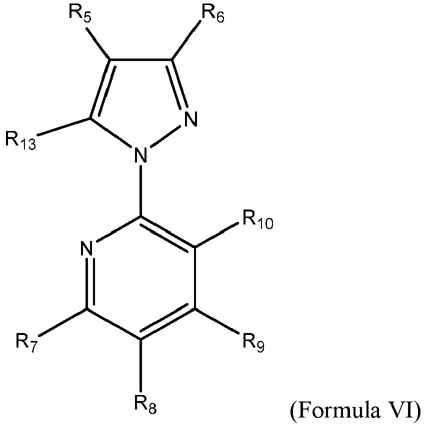
   each of R₅ - R₁₀ is independently selected from hydrogen and halogen; and
   R₁₃ is an organic acid, the method comprising
      I) forming a mixture comprising
         A) a compound of Formula V, wherein 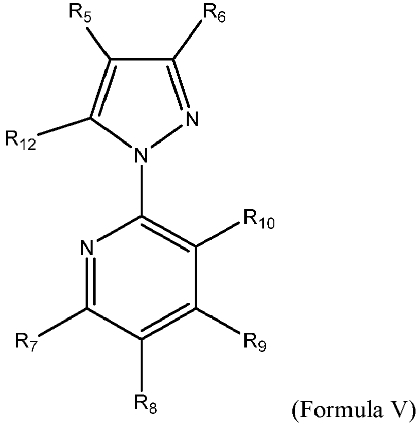
            each of R₅ - R₁₀ is independently selected from hydrogen and halogen;
            R₁₂ is selected from ether, ester, and nitrile; and
            wherein the compound of Formula V is prepared according to a method comprising
               i) forming a mixture comprising
                  a) a compound of Formula III, wherein 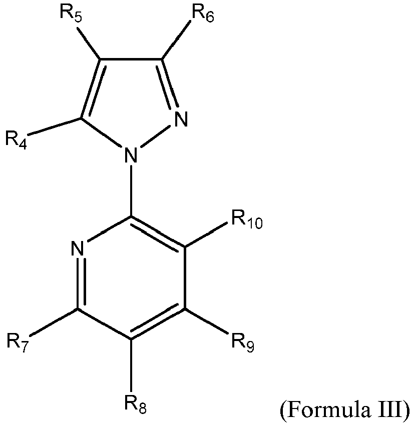
                     R₄ is hydrogen;
                     each of R₅ - R₁₀ is independently selected from hydrogen and halogen; and
                     wherein the compound of Formula III is prepared according to a method comprising
                        IA) forming a mixture comprising
                           AA) a compound of Formula II, wherein 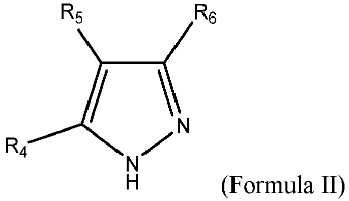
                              each of R₄, R₅, and R₆ is independently selected from hydrogen and halogen;
                              wherein at least one of R₄, R₅, and R₆ is hydrogen; and
                              wherein the compound of Formula II is prepared according to a method comprising
                                 ia) forming a mixture comprising
                                    aa) a compound of Formula VIII, wherein 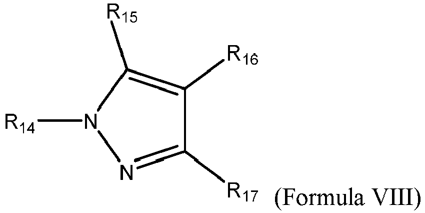
                                       R₁₄ is selected from substituted or unsubstituted carbocycle and substituted or unsubstituted heterocycle;
                                       each of R₁₅, R₁₆, and R₁₇ is independently selected from hydrogen and halogen; and
                                       wherein at least one of R₁₅, R₁₆, and R₁₇ is a halogen;
                                    bb) a solvent; and
                                    cc) an acid; and
                                 iia) reacting the mixture;
                           BB) a compound of Formula IV, wherein 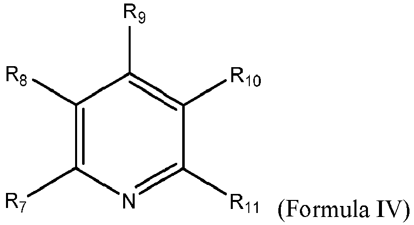 each of R₇ - R₁₁ is independently selected from hydrogen and halogen;
                           CC) a solvent;
                           DD) an inorganic base; and
                           EE) optionally an additive; and
                        IIA) reacting the mixture;
                  b) a solvent;
                  c) a base comprising
                     a compound comprising a metal; and
                     optionally a first amine base;
                  d) optionally an additive; and
                  e) optionally a second amine base; and
               ii) reacting the mixture with an organic compound; and
         B) a metal hydroxide; and
      II) reacting the mixture.
Embodiment 2. The method of embodiment 1, wherein the metal hydroxide is selected from alkali hydroxide, alkaline earth metal hydroxide, and combinations thereof.
Embodiment 3. The method of embodiment 2, wherein the alkali hydroxide is selected from lithium hydroxide, sodium hydroxide, and potassium hydroxide.
Embodiment 4. The method of embodiment 2, wherein the alkaline earth metal hydroxide is selected from calcium hydroxide and barium hydroxide.
Embodiment 5. The method of embodiment 1, wherein the method step II) of reacting the mixture occurs at a reaction temperature in the range of about 0 °C to about 90 °C.
Embodiment 6. The method of embodiment 1, wherein the compound comprising a metal is a Grignard reagent.
Embodiment 7. The method of embodiment 1, wherein the compound comprising a metal is selected from *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, MeMgCl, MeMgBr, *i*PrMgCl, *i*PrMgBr, nBuMgCl, nBuMgBr, tBuMgCl, and combinations thereof.
Embodiment 8. The method of embodiment 1, wherein the first amine base and the second amine base are each independently selected from *i*Pr₂NH, TMP, hexamethyldisilazane, Et₂NH, c-hexyl₂NH, and combinations thereof.
Embodiment 9. The method of embodiment 1, wherein at least one of the first amine base and the second amine base are present in a stoichiometric or catalytic amount relative to the compound comprising a metal.
Embodiment 10. The method of embodiment 1 wherein the base is *i*Pr₂NMgCl.
Embodiment 11. The method of embodiment 1 wherein the base comprises MeMgCl and *i*Pr₂NH.
Embodiment 12. The method of embodiment 1, wherein the solvent b) is selected from THF, toluene, 1,4-dioxane, Me-THF, and combinations thereof.
Embodiment 13. The method of embodiment 1, wherein the organic compound is selected from dimethyl carbonate, N,N-dimethyacetamide, and combinations thereof.
Embodiment 14. The method of embodiment 13, wherein the organic compound is dimethyl carbonate.
Embodiment 15. The method of embodiment 1, wherein the additive d) is selected from LiCl, TMEDA, and combinations thereof.
Embodiment 16. The method of embodiment 1, wherein the method step ii) of reacting the mixture occurs at a reaction temperature in the range of about 0 °C to about 50 °C.
Embodiment 17. The method of embodiment 16, wherein the method step ii) of reacting the mixture occurs at a reaction temperature in the range of about 0 °C to about 30 °C.
Embodiment 18. The method of embodiment 17, wherein the method step ii) of reacting the mixture occurs at a reaction temperature in the range of about 10 °C to about 25 °C.
Embodiment 19. The method of embodiment 1, wherein R₅ and R₆ of Formula III are each independently hydrogen.
Embodiment 20. The method of embodiment 1, wherein the inorganic base DD) is selected from powder sodium hydroxide, powder potassium hydroxide, potassium carbonate, potassium phosphate, powder sodium methoxide, powder potassium *t*-butoxide, and combinations thereof.
Embodiment 21. The method of embodiment 1, wherein the solvent CC) is selected from toluene, N,N-dimethylformamide, N,N-dimethylacetamide, N-methyl-2-pyrrolidone, acetonitrile, and combinations thereof.
Embodiment 22. The method of embodiment 1, wherein the additive EE) is selected from potassium iodide, a phase transfer catalyst, and combinations thereof.
Embodiment 23. The method of embodiment 22, wherein the phase transfer catalyst is selected from butyl ammonium chloride, tetra butyl ammonium bromide, aliquat-336, 18-crown-6, and combinations thereof.
Embodiment 24. The method of embodiment 1, wherein the method step IIA) of reacting the mixture occurs at a reaction temperature in the range of about 100 °C to about 200 °C.
Embodiment 25. The method of embodiment 1, wherein the solvent bb) is selected from methanol, ethanol, isopropanol, toluene, 1,4-dioxane, tetrahydrofuran, and combinations thereof.
Embodiment 26. The method of embodiment 1, wherein the acid cc) is selected from hydrochloric acid, sulfuric acid, phosphoric acid, methylsulfonic acid, trifluoroacetic acid, hydrobromic acid, and combinations thereof.
Embodiment 27. The method of embodiment 1, wherein the method step iia) of reacting the mixture occurs at a reaction temperature in the range of about 20 °C to about 150 °C.
Embodiment 28. The method of embodiment 1, wherein R₁₄ is tetrahydropyranyl.
Embodiment 29. The method of embodiment 1, wherein R₁₂ is selected from ester and nitrile.
Embodiment 30. The method of embodiment 1, wherein the compound of Formula VIII is prepared according to a method comprising
   I) forming a mixture comprising
      A) a compound of Formula VII, wherein 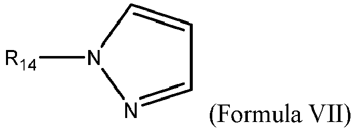 R₁₄ is selected from substituted or unsubstituted carbocycle and substituted or unsubstituted heterocycle;
      B) a solvent;
      C) a base comprising
         a compound comprising a metal; and
         optionally a first amine base; and
      D) optionally a second amine base; and
   II) reacting the mixture with a halogenation reagent.
Embodiment 31. The method of embodiment 30, wherein the halogenation reagent is selected from bromine, N-bromosuccinimide, 1,3-dibromo-5,5-dimethylhylhydantoin, 1,2-dibromotetrachloroethane, tetrabutylammonium tribromide, quaternary ammonium salts of tribromide, 1,2-dibromotrichloroethane, diethyl dibromomalonate, and combinations thereof.
Embodiment 32. The method of embodiment 30, wherein the solvent is selected from THF, toluene, 1,4-dioxane, Me-THF, and combinations thereof.
Embodiment 33. The method of embodiment 30, wherein the compound comprising a metal is a Grignard reagent.
Embodiment 34. The method of embodiment 30, wherein the compound comprising a metal is selected from *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, MeMgCl, MeMgBr, *i*PrMgCl, *i*PrMgBr, nBuMgCl, nBuMgBr, tBuMgCl, and combinations thereof.
Embodiment 35. The method of embodiment 30, wherein the first amine base and the second amine base are each independently selected from *i*Pr₂NH, TMP, Et₂NH, c-hexyl₂NH, and combinations thereof.
Embodiment 36. The method of embodiment 30, wherein at least one of the first amine base and the second amine base are present in an amount relative to the compound comprising a metal in the range of about 1 mol% to about 90 mol%.
Embodiment 37. The method of embodiment 36, wherein at least one of the first amine base and the second amine base are present in an amount relative to the compound comprising a metal in the range of about 5 mol% to about 50 mol%.
Embodiment 38. The method of embodiment 37, wherein at least one of the first amine base and the second amine base are present in an amount relative to the compound comprising a metal in the range of about 10 mol% to about 20 mol%.
Embodiment 39. The method of embodiment 30, wherein the method step of reacting the mixture occurs at a reaction temperature in the range of about 0 °C to about 60 °C.
Embodiment 40. The method of embodiment 39, wherein the method step of reacting the mixture occurs at a reaction temperature in the range of about 40 °C to about 50 °C.
Embodiment 41. The method of embodiment 30, wherein the method produces a mixture of compounds of Formula VIII.
Embodiment 42. The method of embodiment 30, wherein the method produces a compound selected from 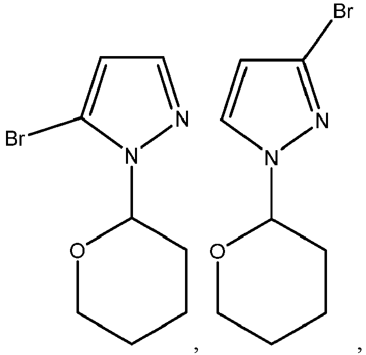 and combinations thereof.
Embodiment 43. The method of embodiment 30, wherein R₁₄ is tetrahydropyranyl.
Embodiment 44. The method of embodiment 30, wherein the compound of Formula VII is prepared according to a method comprising
   I) forming a mixture comprising
      A) pyrazole or a pyrazole derivative;
      B) a cycloalkene or heterocycloalkene;
      C) optionally a solvent; and
      D) an acid; and
   II) reacting the mixture.
Embodiment 45. The method of embodiment 44, wherein the cycloalkene or heterocycloalkene is selected from 3,4-dihydropyran, 3,6-dihydropyran, cyclohexene, and combinations thereof.
Embodiment 46. The method of embodiment 44, wherein the solvent is selected from toluene, xylene, heptanes, and combinations thereof.
Embodiment 47. The method of embodiment 44, wherein the acid is selected from organic acid, inorganic acid, and combinations thereof.
Embodiment 48. The method of embodiment 44, wherein the acid is selected from trifluoroacetic acid, sulfuric acid, p-toluenesulfonic acid, methylsulfonic acid, hydrochloric acid, hydrobromic acid, phosphoric acid, and combinations thereof.
Embodiment 49. The method of embodiment 44, wherein the method step of reacting the mixture occurs at a reaction temperature in the range of about 25 °C to about 120 °C.
Embodiment 50. The method of embodiment 49, wherein the method step of reacting the mixture occurs at a reaction temperature in the range of about 60 °C to about 95 °C.
Embodiment 51. A method of preparing a compound of Formula VI, wherein 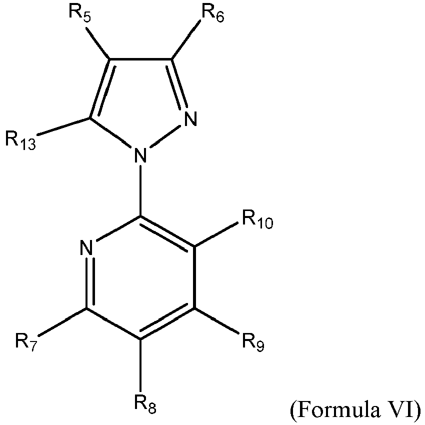
   each of R₅ - R₁₀ is independently selected from hydrogen and halogen; and
   R₁₃ is an organic acid, the method comprising
      I) forming a mixture comprising
         A) a compound of Formula III, wherein 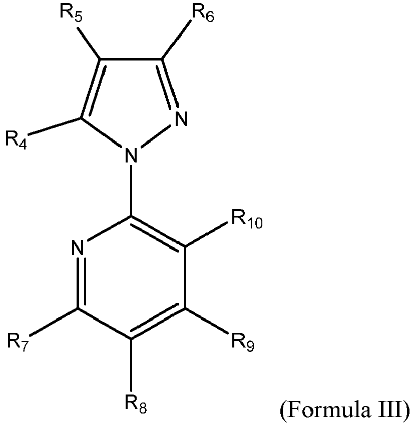
            R₄ is hydrogen;
            each of R₅ - R₁₀ is independently selected from hydrogen and halogen; and
            wherein the compound of Formula III is prepared according to a method comprising
               i) forming a mixture comprising
                  a) a compound of Formula II, wherein 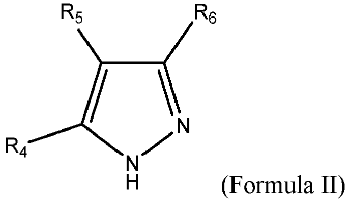 each of R₄, R₅, and R₆ is independently selected from hydrogen
                     and halogen;
                     wherein at least one of R₄, R₅, and R₆ is hydrogen; and wherein the compound of Formula II is prepared according to a method comprising
                     IA) forming a mixture comprising
                        AA) a compound of Formula VIII, wherein 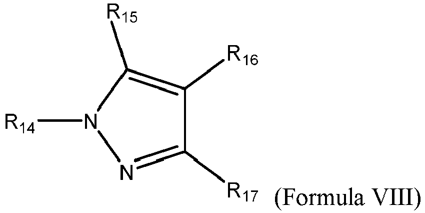
                           R₁₄ is selected from substituted or unsubstituted carbocycle and substituted or unsubstituted heterocycle;
                           each of R₁₅, R₁₆, and R₁₇ is independently selected from hydrogen and halogen; and
                           wherein at least one of R₁₅, R₁₆, and R₁₇ is a halogen;
                        BB) a solvent; and
                        CC) an acid; and
                     IIa) reacting the mixture;
                  b) a compound of Formula IV, wherein 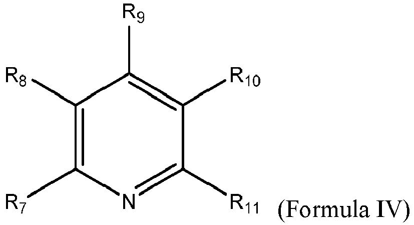 each of R₇ - R₁₁ is independently selected from hydrogen and halogen;
                  c) a solvent;
                  d) an inorganic base; and
                  e) optionally an additive; and
               ii) reacting the mixture;
         B) a carbonyl-containing compound;
         C) a solvent;
         D) a base comprising
            a compound comprising a metal; and
            optionally a first amine base;
         E) optionally an additive; and
         F) optionally a second amine base; and
      II) reacting the mixture.
Embodiment 52. The method of embodiment 51, wherein the compound comprising a metal is a Grignard reagent.
[0089] Embodiment 53. The method of embodiment 51, wherein the compound comprising a metal is selected from *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, MeMgCl, MeMgBr, *i*PrMgCl, *i*PrMgBr, nBuMgCl, nBuMgBr, tBuMgCl, and combinations thereof.
Embodiment 54. The method of embodiment 51, wherein the first amine base and the second amine base are each independently selected from *i*Pr₂NH, TMP, hexamethyldisilazane, Et₂NH, c-hexyl₂NH, and combinations thereof.
Embodiment 55. The method of embodiment 51, wherein at least one of the first amine base and the second amine base are present in a stoichiometric or catalytic amount relative to the compound comprising a metal.
Embodiment 56. The method of embodiment 51 wherein the base is *i*Pr₂NMgCl.
Embodiment 57. The method of embodiment 51 wherein the base comprises MeMgCl and *i*Pr₂NH.
Embodiment 58. The method of embodiment 51, wherein the solvent C) is selected from THF, toluene, 1,4-dioxane, Me-THF, and combinations thereof.
Embodiment 59. The method of embodiment 51, wherein the carbonyl-containing compound is selected from dimethylcarbonate, N,N-dimethylacetamide, carbon dioxide, and combinations thereof.
Embodiment 60. The method of embodiment 59, wherein the carbonyl-containing compound is carbon dioxide.
Embodiment 61. The method of embodiment 51, wherein the additive E) is selected from LiCl, TMEDA, and combinations thereof.
Embodiment 62. The method of embodiment 51, wherein the method step II) of reacting the mixture occurs at a reaction temperature in the range of about 0 °C to about 50 °C.
Embodiment 63. The method of embodiment 62, wherein the method step II) of reacting the mixture occurs at a reaction temperature in the range of about 0 °C to about 30 °C.
Embodiment 64. The method of embodiment 63, wherein the method step II) of reacting the mixture occurs at a reaction temperature in the range of about 10 °C to about 25 °C.
Embodiment 65. The method of embodiment 51, wherein the inorganic base d) is selected from powder sodium hydroxide, powder potassium hydroxide, potassium carbonate, potassium phosphate, powder sodium methoxide, powder potassium t-butoxide, and combinations thereof.
Embodiment 66. The method of embodiment 51, wherein the solvent c) is selected from toluene, N,N-dimethylformamide, N,N-dimethylacetamide, N-methyl-2-pyrrolidone, acetonitrile, and combinations thereof.
Embodiment 67. The method of embodiment 51, wherein the additive e) is selected from potassium iodide, a phase transfer catalyst, and combinations thereof.
Embodiment 68. The method of embodiment 67, wherein the phase transfer catalyst is selected from butyl ammonium chloride, tetra butyl ammonium bromide, aliquat-336, 18-crown-6, and combinations thereof.
Embodiment 69. The method of embodiment 51, wherein the method step ii) of reacting the mixture occurs at a reaction temperature in the range of about 100 °C to about 180 °C.
Embodiment 70. The method of embodiment 51, wherein the solvent BB) is selected from methanol, ethanol, isopropanol, toluene, 1,4-dioxane, tetrahydrofuran, and combinations thereof.
Embodiment 71. The method of embodiment 51, wherein the acid CC) is selected from hydrochloric acid, sulfuric acid, phosphoric acid, methylsulfonic acid, trifluoroacetic acid, hydrobromic acid, and combinations thereof.
Embodiment 72. The method of embodiment 51, wherein the method step IIa) of reacting the mixture occurs at a reaction temperature in the range of about 20 °C to about 150 °C.
Embodiment 73. The method of embodiment 51, wherein the compound of Formula VIII is prepared according to a method comprising
   I) forming a mixture comprising
      A) a compound of Formula VII, wherein 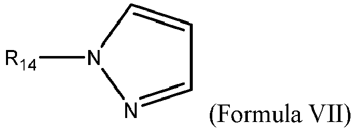 R₁₄ is selected from substituted or unsubstituted carbocycle and substituted or unsubstituted heterocycle;
      B) a solvent;
      C) a base comprising
         a compound comprising a metal; and
         optionally a first amine base; and
      D) optionally a second amine base; and
   II) reacting the mixture with a halogenation reagent.
Embodiment 74. The method of embodiment 73, wherein the halogenation reagent is selected from bromine, N-bromosuccinimide, 1,3-dibromo-5,5-dimethylhylhydantoin, 1,2-dibromotetrachloroethane, tetrabutylammonium tribromide, quaternary ammonium salts of tribromide, 1,2-dibromotrichloroethane, diethyl dibromomalonate, and combinations thereof.
Embodiment 75. The method of embodiment 73, wherein the solvent is selected from THF, toluene, 1,4-dioxane, Me-THF, and combinations thereof.
Embodiment 76. The method of embodiment 73, wherein the compound comprising a metal is a Grignard reagent.
Embodiment 77. The method of embodiment 73, wherein the compound comprising a metal is selected from *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, MeMgCl, MeMgBr, *i*PrMgCl, *i*PrMgBr, nBuMgCl, nBuMgBr, tBuMgCl, and combinations thereof.
Embodiment 78. The method of embodiment 73, wherein the first amine base and the second amine base are each independently selected from *i*Pr₂NH, TMP, Et₂NH, c-hexyl₂NH, and combinations thereof.
Embodiment 79. The method of embodiment 73, wherein at least one of the first amine base and the second amine base are present in an amount relative to the compound comprising a metal in the range of about 1 mol% to about 90 mol%.
Embodiment 80. The method of embodiment 79, wherein at least one of the first amine base and the second amine base are present in an amount relative to the compound comprising a metal in the range of about 5 mol% to about 50 mol%.
Embodiment 81. The method of embodiment 80, wherein at least one of the first amine base and the second amine base are present in an amount relative to the compound comprising a metal in the range of about 10 mol% to about 20 mol%.
Embodiment 82. The method of embodiment 73, wherein the method step of reacting the mixture occurs at a reaction temperature in the range of about 0 °C to about 60 °C.
Embodiment 83. The method of embodiment 82, wherein the method step of reacting the mixture occurs at a reaction temperature in the range of about 40 °C to about 50 °C.
Embodiment 84. The method of embodiment 73, wherein the method produces a mixture of compounds of Formula VIII.
Embodiment 85. The method of embodiment 73, wherein the method produces a compound selected from 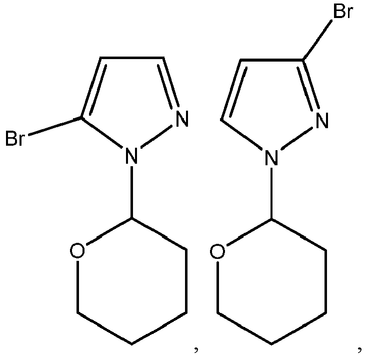 and combinations thereof.
Embodiment 86. The method of embodiment 73, wherein R₁₄ is tetrahydropyranyl.
Embodiment 87. The method of embodiment 73, wherein the compound of Formula VII is prepared according to a method comprising
   I) forming a mixture comprising
      A) pyrazole or a pyrazole derivative;
      B) a cycloalkene or heterocycloalkene;
      C) optionally a solvent; and
      D) an acid; and
   II) reacting the mixture.
Embodiment 88. The method of embodiment 87, wherein the cycloalkene or heterocycloalkene is selected from 3,4-dihydropyran, 3,6-dihydropyran, cyclohexene, and combinations thereof.
Embodiment 89. The method of embodiment 87, wherein the solvent is selected from toluene, xylene, heptanes, and combinations thereof.
Embodiment 90. The method of embodiment 87, wherein the acid is selected from organic acid, inorganic acid, and combinations thereof.
Embodiment 91. The method of embodiment 87, wherein the acid is selected from trifluoroacetic acid, sulfuric acid, p-toluenesulfonic acid, methylsulfonic acid, hydrochloric acid, hydrobromic acid, phosphoric acid, and combinations thereof.
Embodiment 92. The method of embodiment 87, wherein the method step of reacting the mixture occurs at a reaction temperature in the range of about 25 °C to about 120 °C.
Embodiment 93. The method of embodiment 92, wherein the method step of reacting the mixture occurs at a reaction temperature in the range of about 60 °C to about 95 °C.
Embodiment 94. A method of preparing a compound of Formula II, wherein 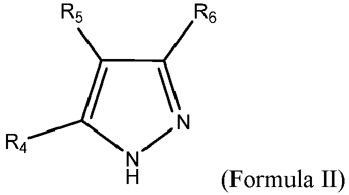
   each of R₄, R₅, and R₆ is independently selected from hydrogen and halogen; and
   wherein at least one of R₄, R₅, and R₆ is hydrogen, the method comprising
      I) forming a mixture comprising
         A) a compound of Formula VIII, wherein 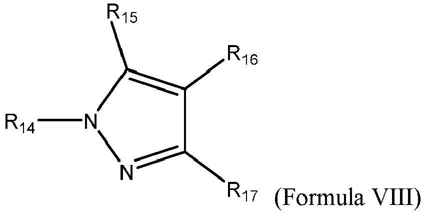
            R₁₄ is selected from substituted or unsubstituted carbocycle and substituted or unsubstituted heterocycle;
            each of R₁₅, R₁₆, and R₁₇ is independently selected from hydrogen and halogen; and
            wherein at least one of R₁₅, R₁₆, and R₁₇ is a halogen;
            B) a solvent; and
            C) an acid; and
      II) reacting the mixture.
Embodiment 95. The method of embodiment 94, wherein the solvent is selected from methanol, ethanol, isopropanol, toluene, 1,4-dioxane, tetrahydrofuran, and combinations thereof.
Embodiment 96. The method of embodiment 94, wherein the acid is selected from hydrochloric acid, sulfuric acid, phosphoric acid, methylsulfonic acid, trifluoroacetic acid, hydrobromic acid, and combinations thereof.
Embodiment 97. The method of embodiment 94, wherein the method step of reacting the mixture occurs at a reaction temperature in the range of about 20 °C to about 150 °C.
Embodiment 98. The method of embodiment 94, wherein the compound of Formula VIII is prepared according to a method comprising
   I) forming a mixture comprising
      A) a compound of Formula VII, wherein 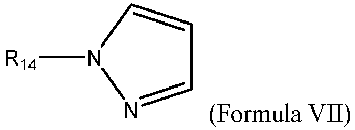 R₁₄ is selected from substituted or unsubstituted carbocycle and substituted or unsubstituted heterocycle;
      B) a solvent;
      C) a base comprising
         a compound comprising a metal; and
         optionally a first amine base; and
      D) optionally a second amine base; and
   II) reacting the mixture with a halogenation reagent.
Embodiment 99. The method of embodiment 98, wherein the halogenation reagent is selected from bromine, N-bromosuccinimide, 1,3-dibromo-5,5-dimethylhylhydantoin, 1,2-dibromotetrachloroethane, tetrabutylammonium tribromide, quaternary ammonium salts of tribromide, 1,2-dibromotrichloroethane, diethyl dibromomalonate, and combinations thereof.
Embodiment 100. The method of embodiment 98, wherein the solvent is selected from THF, toluene, 1,4-dioxane, Me-THF, and combinations thereof.
Embodiment 101. The method of embodiment 98, wherein the compound comprising a metal is a Grignard reagent.
Embodiment 102. The method of embodiment 98, wherein the compound comprising a metal is selected from *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, MeMgCl, MeMgBr, *i*PrMgCl, *i*PrMgBr, nBuMgCl, nBuMgBr, tBuMgCl, and combinations thereof.
Embodiment 103. The method of embodiment 98, wherein the first amine base and the second amine base are each independently selected from *i*Pr₂NH, TMP, Et₂NH, c-hexyl₂NH, and combinations thereof.
Embodiment 104. The method of embodiment 98, wherein at least one of the first amine base and the second amine base are present in an amount relative to the compound comprising a metal in the range of about 1 mol% to about 90 mol%.
Embodiment 105. The method of embodiment 104, wherein at least one of the first amine base and the second amine base are present in an amount relative to the compound comprising a metal in the range of about 5 mol% to about 50 mol%.
Embodiment 106. The method of embodiment 105, wherein at least one of the first amine base and the second amine base are present in an amount relative to the compound comprising a metal in the range of about 10 mol% to about 20 mol%.
Embodiment 107. The method of embodiment 98, wherein the method step of reacting the mixture occurs at a reaction temperature in the range of about 0 °C to about 60 °C.
Embodiment 108. The method of embodiment 107, wherein the method step of reacting the mixture occurs at a reaction temperature in the range of about 40 °C to about 50 °C.
Embodiment 109. The method of embodiment 98, wherein the method produces a mixture of compounds of Formula VIII.
Embodiment 110. The method of embodiment 98, wherein the method produces a compound selected from 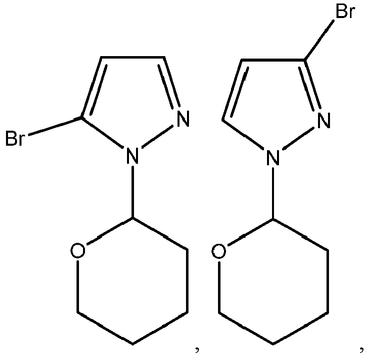 and combinations thereof.
Embodiment 111. The method of embodiment 98, wherein R₁₄ is tetrahydropyranyl.
Embodiment 112. The method of embodiment 98, wherein the compound of Formula VII is prepared according to a method comprising
   I) forming a mixture comprising
      A) pyrazole or a pyrazole derivative;
      B) a cycloalkene or heterocycloalkene;
      C) optionally a solvent; and
      D) an acid; and
   II) reacting the mixture.
Embodiment 113. The method of embodiment 112, wherein the cycloalkene or heterocycloalkene is selected from 3,4-dihydropyran, 3,6-dihydropyran, cyclohexene, and combinations thereof.
Embodiment 114. The method of embodiment 112, wherein the solvent is selected from toluene, xylene, heptanes, and combinations thereof.
Embodiment 115. The method of embodiment 112, wherein the acid is selected from organic acid, inorganic acid, and combinations thereof.
Embodiment 116. The method of embodiment 112, wherein the acid is selected from trifluoroacetic acid, sulfuric acid, p-toluenesulfonic acid, methylsulfonic acid, hydrochloric acid, hydrobromic acid, phosphoric acid, and combinations thereof.
Embodiment 117. The method of embodiment 112, wherein the method step of reacting the mixture occurs at a reaction temperature in the range of about 25 °C to about 120 °C.
Embodiment 118. The method of embodiment 117, wherein the method step of reacting the mixture occurs at a reaction temperature in the range of about 60 °C to about 95 °C.
Embodiment 119. A method of preparing a compound of Formula VII, wherein 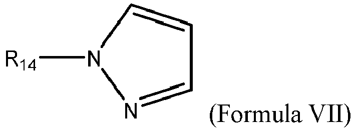 R₁₄ is selected from substituted or unsubstituted carbocycle and substituted or unsubstituted heterocycle, the method comprising
   I) forming a mixture comprising
      A) pyrazole or a pyrazole derivative;
      B) a cycloalkene or heterocycloalkene;
      C) optionally a solvent; and
      D) an acid; and
   II) reacting the mixture.
Embodiment 120. The method of embodiment 119, wherein the cycloalkene or heterocycloalkene is selected from 3,4-dihydropyran, 3,6-dihydropyran, cyclohexene, and combinations thereof.
Embodiment 121. The method of embodiment 119, wherein the solvent is selected from toluene, xylene, heptanes, and combinations thereof.
Embodiment 122. The method of embodiment 119, wherein the acid is selected from organic acid, inorganic acid, and combinations thereof.
Embodiment 123. The method of embodiment 119, wherein the acid is selected from trifluoroacetic acid, sulfuric acid, p-toluenesulfonic acid, methylsulfonic acid, hydrochloric acid, hydrobromic acid, phosphoric acid, and combinations thereof.
Embodiment 124. The method of embodiment 119, wherein the method step of reacting the mixture occurs at a reaction temperature in the range of about 25 °C to about 120 °C.
Embodiment 125. The method of embodiment 124, wherein the method step of reacting the mixture occurs at a reaction temperature in the range of about 60 °C to about 95 °C.
Embodiment 126. A method of preparing a compound of Formula VIII, wherein 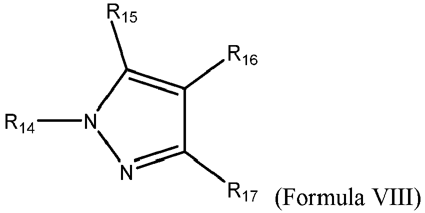
   R₁₄ is selected from substituted or unsubstituted carbocycle and substituted or unsubstituted heterocycle;
   each of R₁₅, R₁₆, and R₁₇ is independently selected from hydrogen and halogen; and
   wherein at least one of R₁₅, R₁₆, and R₁₇ is a halogen, the method comprising
      I) forming a mixture comprising
         A) the compound of Formula VII, wherein 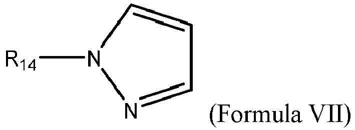 R₁₄ is selected from substituted or unsubstituted carbocycle and substituted or unsubstituted heterocycle;
         B) a solvent;
         C) a base comprising
            a compound comprising a metal; and
            optionally a first amine base; and
         D) optionally a second amine base; and
      II) reacting the mixture with a halogenation reagent.
Embodiment 127. The method of embodiment 126, wherein the halogenation reagent is selected from bromine, N-bromosuccinimide, 1,3-dibromo-5,5-dimethylhylhydantoin, 1,2-dibromotetrachloroethane, tetrabutylammonium tribromide, quaternary ammonium salts of tribromide, 1,2-dibromotrichloroethane, diethyl dibromomalonate, and combinations thereof.
Embodiment 128. The method of embodiment 126, wherein the solvent is selected from THF, toluene, 1,4-dioxane, Me-THF, and combinations thereof.
Embodiment 129. The method of embodiment 126, wherein the compound comprising a metal is a Grignard reagent.
Embodiment 130. The method of embodiment 126, wherein the compound comprising a metal is selected from *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, MeMgCl, MeMgBr, *i*PrMgCl, *i*PrMgBr, nBuMgCl, nBuMgBr, tBuMgCl, and combinations thereof.
Embodiment 131. The method of embodiment 126, wherein the first amine base and the second amine base are each independently selected from *i*Pr₂NH, TMP, Et₂NH, c-hexyl₂NH, and combinations thereof.
Embodiment 132. The method of embodiment 126, wherein at least one of the first amine base and the second amine base are present in an amount relative to the compound comprising a metal in the range of about 1 mol% to about 90 mol%.
Embodiment 133. The method of embodiment 132, wherein at least one of the first amine base and the second amine base are present in an amount relative to the compound comprising a metal in the range of about 5 mol% to about 50 mol%.
Embodiment 134. The method of embodiment 133, wherein at least one of the first amine base and the second amine base are present in an amount relative to the compound comprising a metal in the range of about 10 mol% to about 20 mol%.
Embodiment 135. The method of embodiment 126, wherein the method step of reacting the mixture occurs at a reaction temperature in the range of about 0 °C to about 60 °C.
Embodiment 136. The method of embodiment 135, wherein the method step of reacting the mixture occurs at a reaction temperature in the range of about 40 °C to about 50 °C.
Embodiment 137. The method of embodiment 126, wherein the method produces a mixture of compounds of Formula VIII.
Embodiment 138. The method of embodiment 126, wherein the method produces a compound selected from 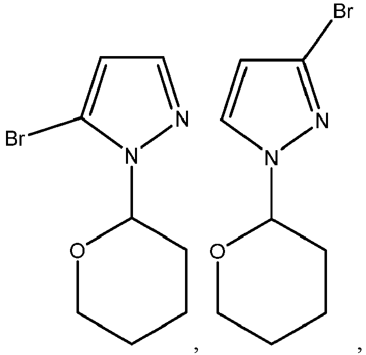 and combinations thereof.
Embodiment 139. The method of embodiment 126, wherein R₁₄ is tetrahydropyranyl.
Embodiment 140. The method of embodiment 126, wherein the compound of Formula VII is prepared according to a method comprising
   I) forming a mixture comprising
      A) pyrazole or a pyrazole derivative;
      B) a cycloalkene or heterocycloalkene;
      C) optionally a solvent; and
      D) an acid; and
   II) reacting the mixture.
Embodiment 141. The method of embodiment 140, wherein the cycloalkene or heterocycloalkene is selected from 3,4-dihydropyran, 3,6-dihydropyran, cyclohexene, and combinations thereof.
Embodiment 142. The method of embodiment 140, wherein the solvent is selected from toluene, xylene, heptanes, and combinations thereof.
Embodiment 143. The method of embodiment 140, wherein the acid is selected from organic acid, inorganic acid, and combinations thereof.
Embodiment 144. The method of embodiment 140, wherein the acid is selected from trifluoroacetic acid, sulfuric acid, p-toluenesulfonic acid, methylsulfonic acid, hydrochloric acid, hydrobromic acid, phosphoric acid, and combinations thereof.
Embodiment 145. The method of embodiment 140, wherein the method step of reacting the mixture occurs at a reaction temperature in the range of about 25 °C to about 120 °C.
Embodiment 146. The method of embodiment 145, wherein the method step of reacting the mixture occurs at a reaction temperature in the range of about 60 °C to about 95 °C.
Embodiment 147. A method of preparing a compound of Formula V, wherein 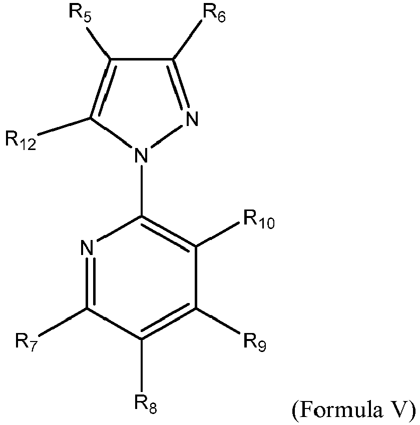
   each of R₅ - R₁₀ is independently selected from hydrogen and halogen; and
   R₁₂ is selected from ether, ester, and nitrile, the method comprising
      i) forming a mixture comprising
         a) a compound of Formula III, wherein 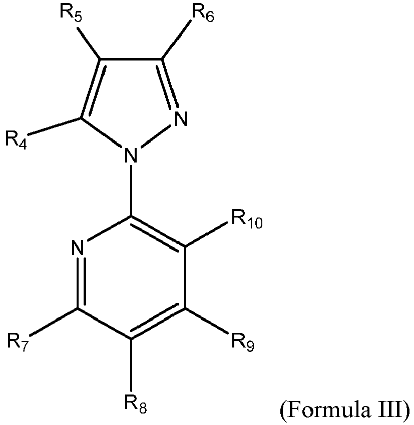
            R₄ is hydrogen;
            each of R₅ - R₁₀ is independently selected from hydrogen and halogen; and
            wherein the compound of Formula III is prepared according to a method comprising
               IA) forming a mixture comprising
                  AA) a compound of Formula II, wherein 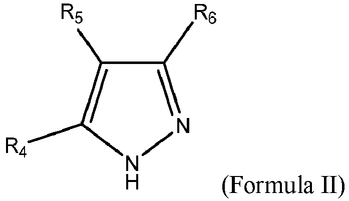
                     each of R₄, R₅, and R₆ is independently selected from hydrogen and halogen;
                     wherein at least one of R₄, R₅, and R₆ is hydrogen; and
                     wherein the compound of Formula II is prepared according to a method comprising
                        ia) forming a mixture comprising
                           aa) a compound of Formula VIII, wherein 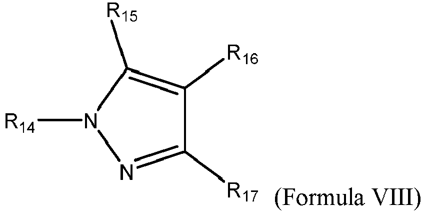
                              R₁₄ is selected from substituted or unsubstituted carbocycle and substituted or unsubstituted heterocycle;
                              each of R₁₅, R₁₆, and R₁₇ is independently selected from hydrogen and halogen; and
                              wherein at least one of R₁₅, R₁₆, and R₁₇ is a halogen;
                           bb) a solvent; and
                           cc) an acid; and
                        iia) reacting the mixture;
                  BB) a compound of Formula IV, wherein 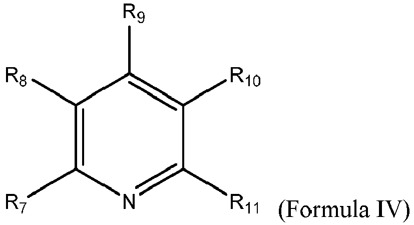 each of R₇ - R₁₁ is independently selected from hydrogen and halogen;
                  CC) a solvent;
                  DD) an inorganic base; and
                  EE) optionally an additive; and
               IIA) reacting the mixture;
         b) a solvent;
         c) a base comprising
            a compound comprising a metal; and
            optionally a first amine base;
         d) optionally an additive; and
         e) optionally a second amine base; and
      ii) reacting the mixture with an organic compound.
Embodiment 148. The method of embodiment 147, wherein the compound comprising a metal is a Grignard reagent.
Embodiment 149. The method of embodiment 147, wherein the compound comprising a metal is selected from *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, MeMgCl, MeMgBr, iPrMgCl, iPrMgBr, nBuMgCl, nBuMgBr, tBuMgCl, and combinations thereof.
Embodiment 150. The method of embodiment 147, wherein the first amine base and the second amine base are each independently selected from *i*Pr₂NH, TMP, hexamethyldisilazane, Et₂NH, c-hexyl₂NH, and combinations thereof.
Embodiment 151. The method of embodiment 147, wherein at least one of the first amine base and the second amine base are present in a stoichiometric or catalytic amount relative to the compound comprising a metal.
Embodiment 152. The method of embodiment 147 wherein the base is *i*Pr₂NMgCl.
Embodiment 153. The method of embodiment 147 wherein the base comprises MeMgCl and *i*Pr₂NH.
Embodiment 154. The method of embodiment 147, wherein the solvent b) is selected from THF, toluene, 1,4-dioxane, Me-THF, and combinations thereof.
Embodiment 155. The method of embodiment 147, wherein the organic compound is selected from dimethyl carbonate, N,N-dimethyacetamide, and combinations thereof.
Embodiment 156. The method of embodiment 155, wherein the organic compound is dimethyl carbonate.
Embodiment 157. The method of embodiment 147, wherein the additive d) is selected from LiCl, TMEDA, and combinations thereof.
Embodiment 158. The method of embodiment 147, wherein the method step ii) of reacting the mixture occurs at a reaction temperature in the range of about 0 °C to about 50 °C.
Embodiment 159. The method of embodiment 158, wherein the method step ii) of reacting the mixture occurs at a reaction temperature in the range of about 0 °C to about 30 °C.
Embodiment 160. The method of embodiment 159, wherein the method step ii) of reacting the mixture occurs at a reaction temperature in the range of about 10 °C to about 25 °C.
Embodiment 161. The method of embodiment 147, wherein R₅ and R₆ of Formula III are each independently hydrogen.
Embodiment 162. The method of embodiment 147, wherein the inorganic base DD) is selected from powder sodium hydroxide, powder potassium hydroxide, potassium carbonate, potassium phosphate, powder sodium methoxide, powder potassium t-butoxide, and combinations thereof.
Embodiment 163. The method of embodiment 147, wherein the solvent CC) is selected from toluene, N,N-dimethylformamide, N,N-dimethylacetamide, N-methyl-2-pyrrolidone, acetonitrile, and combinations thereof.
Embodiment 164. The method of embodiment 147, wherein the additive EE) is selected from potassium iodide, a phase transfer catalyst, and combinations thereof.
Embodiment 165. The method of embodiment 164, wherein the phase transfer catalyst is selected from butyl ammonium chloride, tetra butyl ammonium bromide, aliquat-336, 18-crown-6, and combinations thereof.
Embodiment 166. The method of embodiment 147, wherein the method step IIA) of reacting the mixture occurs at a reaction temperature in the range of about 100 °C to about 200 °C.
Embodiment 167. The method of embodiment 147, wherein the solvent bb) is selected from methanol, ethanol, isopropanol, toluene, 1,4-dioxane, tetrahydrofuran, and combinations thereof.
Embodiment 168. The method of embodiment 147, wherein the acid cc) is selected from hydrochloric acid, sulfuric acid, phosphoric acid, methylsulfonic acid, trifluoroacetic acid, hydrobromic acid, and combinations thereof.
Embodiment 169. The method of embodiment 147, wherein the method step iia) of reacting the mixture occurs at a reaction temperature in the range of about 20 °C to about 150 °C.
Embodiment 170. The method of embodiment 147, wherein R₁₂ is selected from ester and nitrile.
Embodiment 171. The method of embodiment 147, wherein the compound of Formula VIII is prepared according to a method comprising
   I) forming a mixture comprising
      A) a compound of Formula VII, wherein 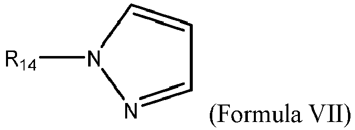 R₁₄ is selected from substituted or unsubstituted carbocycle and substituted or unsubstituted heterocycle;
      B) a solvent;
      C) a base comprising
         a compound comprising a metal; and
         optionally a first amine base; and
      D) optionally a second amine base; and
   II) reacting the mixture with a halogenation reagent.
Embodiment 172. The method of embodiment 171, wherein the halogenation reagent is selected from bromine, N-bromosuccinimide, 1,3-dibromo-5,5-dimethylhylhydantoin, 1,2-dibromotetrachloroethane, tetrabutylammonium tribromide, quaternary ammonium salts of tribromide, 1,2-dibromotrichloroethane, diethyl dibromomalonate, and combinations thereof.
Embodiment 173. The method of embodiment 171, wherein the solvent is selected from THF, toluene, 1,4-dioxane, Me-THF, and combinations thereof.
Embodiment 174. The method of embodiment 171, wherein the compound comprising a metal is a Grignard reagent.
Embodiment 175. The method of embodiment 171, wherein the compound comprising a metal is selected from *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, MeMgCl, MeMgBr, iPrMgCl, iPrMgBr, nBuMgCl, nBuMgBr, tBuMgCl, and combinations thereof.
Embodiment 176. The method of embodiment 171, wherein the first amine base and the second amine base are each independently selected from *i*Pr₂NH, TMP, Et₂NH, c-hexyl₂NH, and combinations thereof.
Embodiment 177. The method of embodiment 171, wherein at least one of the first amine base and the second amine base are present in an amount relative to the compound comprising a metal in the range of about 1 mol% to about 90 mol%.
Embodiment 178. The method of embodiment 177, wherein at least one of the first amine base and the second amine base are present in an amount relative to the compound comprising a metal in the range of about 5 mol% to about 50 mol%.
Embodiment 179. The method of embodiment 178, wherein at least one of the first amine base and the second amine base are present in an amount relative to the compound comprising a metal in the range of about 10 mol% to about 20 mol%.
Embodiment 180. The method of embodiment 171, wherein the method step of reacting the mixture occurs at a reaction temperature in the range of about 0 °C to about 60 °C.
Embodiment 181. The method of embodiment 180, wherein the method step of reacting the mixture occurs at a reaction temperature in the range of about 40 °C to about 50 °C.
Embodiment 182. The method of embodiment 171, wherein the method produces a mixture of compounds of Formula VIII.
Embodiment 183. The method of embodiment 171, wherein the method produces a compound selected from 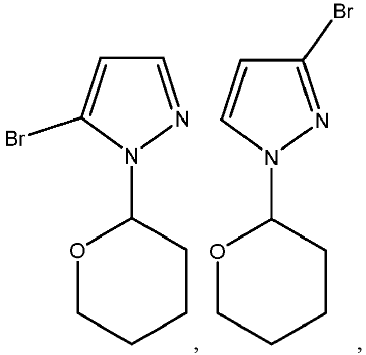 and combinations thereof.
Embodiment 184. The method of embodiment 171, wherein R₁₄ is tetrahydropyranyl.
Embodiment 185. The method of embodiment 171, wherein the compound of Formula VII is prepared according to a method comprising
   I) forming a mixture comprising
      A) pyrazole or a pyrazole derivative;
      B) a cycloalkene or heterocycloalkene;
      C) optionally a solvent; and
      D) an acid; and
   II) reacting the mixture.
Embodiment 186. The method of embodiment 185, wherein the cycloalkene or heterocycloalkene is selected from 3,4-dihydropyran, 3,6-dihydropyran, cyclohexene, and combinations thereof.
Embodiment 187. The method of embodiment 185, wherein the solvent is selected from toluene, xylene, heptanes, and combinations thereof.
Embodiment 188. The method of embodiment 185, wherein the acid is selected from organic acid, inorganic acid, and combinations thereof.
Embodiment 189. The method of embodiment 188, wherein the acid is selected from trifluoroacetic acid, sulfuric acid, p-toluenesulfonic acid, methylsulfonic acid, hydrochloric acid, hydrobromic acid, phosphoric acid, and combinations thereof.
Embodiment 190. The method of embodiment 185, wherein the method step of reacting the mixture occurs at a reaction temperature in the range of about 25 °C to about 120 °C.
Embodiment 191. The method of embodiment 190, wherein the method step of reacting the mixture occurs at a reaction temperature in the range of about 60 °C to about 95 °C.
Embodiment 192. A method of preparing a compound of Formula III, wherein 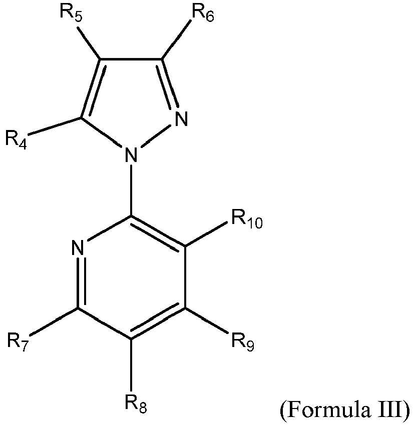
   R₄ is hydrogen; and
   each of R₅ - R₁₀ is independently selected from hydrogen and halogen, the method comprising
      i) forming a mixture comprising
         a) a compound of Formula II, wherein 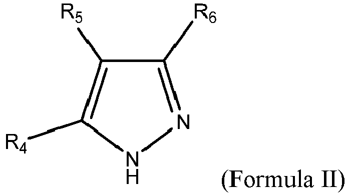
            each of R₄, R₅, and R₆ is independently selected from hydrogen and halogen;
            wherein at least one of R₄, Rs, and R₆ is hydrogen; and
            wherein the compound of Formula II is prepared according to a method comprising
               IA) forming a mixture comprising
                  AA) a compound of Formula VIII, wherein 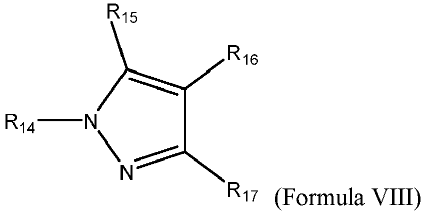
                     R₁₄ is selected from substituted or unsubstituted carbocycle and substituted or unsubstituted heterocycle;
                     each of R₁₅, R₁₆, and R₁₇ is independently selected from hydrogen and halogen; and
                     wherein at least one of R₁₅, R₁₆, and R₁₇ is a halogen;
                  BB) a solvent; and
                  CC) an acid; and
               IIa) reacting the mixture;
         b) a compound of Formula IV, wherein 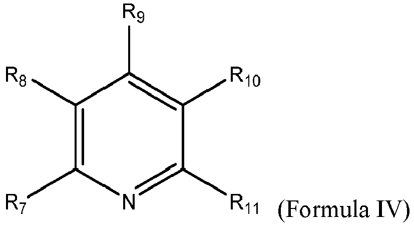 each of R₇ - R₁₁ is independently selected from hydrogen and halogen;
         c) a solvent;
         d) an inorganic base; and
         e) optionally an additive; and
      ii) reacting the mixture.
Embodiment 193. The method of embodiment 192, wherein R₅ and R₆ of Formula III are each independently hydrogen.
Embodiment 194. The method of embodiment 192, wherein the inorganic base d) is selected from powder sodium hydroxide, powder potassium hydroxide, potassium carbonate, potassium phosphate, powder sodium methoxide, powder potassium t-butoxide, and combinations thereof.
Embodiment 195. The method of embodiment 192, wherein the solvent c) is selected from toluene, N,N-dimethylformamide, N,N-dimethylacetamide, N-methyl-2-pyrrolidone, acetonitrile, and combinations thereof.
Embodiment 196. The method of embodiment 192, wherein the additive e) is selected from potassium iodide, a phase transfer catalyst, and combinations thereof.
Embodiment 197. The method of embodiment 196, wherein the phase transfer catalyst is selected from butyl ammonium chloride, tetra butyl ammonium bromide, aliquat-336, 18-crown-6, and combinations thereof.
Embodiment 198. The method of embodiment 192, wherein the method step ii) of reacting the mixture occurs at a reaction temperature in the range of about 100 °C to about 180 °C.
Embodiment 199. The method of embodiment 192, wherein the solvent BB) is selected from methanol, ethanol, isopropanol, toluene, 1,4-dioxane, tetrahydrofuran, and combinations thereof.
Embodiment 200. The method of embodiment 192, wherein the acid CC) is selected from hydrochloric acid, sulfuric acid, phosphoric acid, methylsulfonic acid, trifluoroacetic acid, hydrobromic acid, and combinations thereof.
Embodiment 201. The method of embodiment 192, wherein the method step IIa) of reacting the mixture occurs at a reaction temperature in the range of about 20 °C to about 150 °C.
Embodiment 202. The method of embodiment 192, wherein the compound of Formula VIII is prepared according to a method comprising
   I) forming a mixture comprising
      A) a compound of Formula VII, wherein 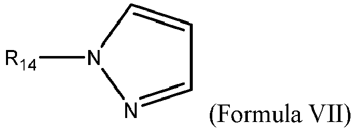 R₁₄ is selected from substituted or unsubstituted carbocycle and substituted or unsubstituted heterocycle;
      B) a solvent;
      C) a base comprising
         a compound comprising a metal; and
         optionally a first amine base; and
      D) optionally a second amine base; and
   II) reacting the mixture with a halogenation reagent.
Embodiment 203. The method of embodiment 202, wherein the halogenation reagent is selected from bromine, N-bromosuccinimide, 1,3-dibromo-5,5-dimethylhylhydantoin, 1,2-dibromotetrachloroethane, tetrabutylammonium tribromide, quaternary ammonium salts of tribromide, 1,2-dibromotrichloroethane, diethyl dibromomalonate, and combinations thereof.
Embodiment 204. The method of embodiment 202, wherein the solvent is selected from THF, toluene, 1,4-dioxane, Me-THF, and combinations thereof.
Embodiment 205. The method of embodiment 202, wherein the compound comprising a metal is a Grignard reagent.
Embodiment 206. The method of embodiment 202, wherein the compound comprising a metal is selected from *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, MeMgCl, MeMgBr, iPrMgCl, iPrMgBr, nBuMgCl, nBuMgBr, tBuMgCl, and combinations thereof.
Embodiment 207. The method of embodiment 202, wherein the first amine base and the second amine base are each independently selected from *i*Pr₂NH, TMP, Et₂NH, c-hexyl₂NH, and combinations thereof.
Embodiment 208. The method of embodiment 202, wherein at least one of the first amine base and the second amine base are present in an amount relative to the compound comprising a metal in the range of about 1 mol% to about 90 mol%.
Embodiment 209. The method of embodiment 208, wherein at least one of the first amine base and the second amine base are present in an amount relative to the compound comprising a metal in the range of about 5 mol% to about 50 mol%.
Embodiment 210. The method of embodiment 209, wherein at least one of the first amine base and the second amine base are present in an amount relative to the compound comprising a metal in the range of about 10 mol% to about 20 mol%.
Embodiment 211. The method of embodiment 202, wherein the method step of reacting the mixture occurs at a reaction temperature in the range of about 0 °C to about 60 °C.
Embodiment 212. The method of embodiment 211, wherein the method step of reacting the mixture occurs at a reaction temperature in the range of about 40 °C to about 50 °C.
Embodiment 213. The method of embodiment 202, wherein the method produces a mixture of compounds of Formula VIII.
Embodiment 214. The method of embodiment 202, wherein the method produces a compound selected from 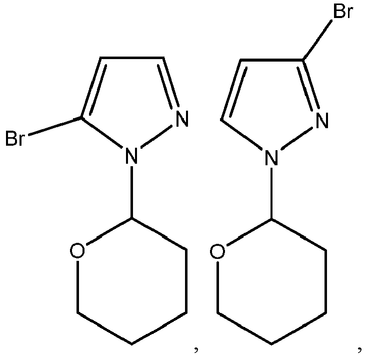 and combinations thereof.
Embodiment 215. The method of embodiment 202, wherein R₁₄ is tetrahydropyranyl.
Embodiment 216. The method of embodiment 202, wherein the compound of Formula VII is prepared according to a method comprising
   I) forming a mixture comprising
      A) pyrazole or a pyrazole derivative;
      B) a cycloalkene or heterocycloalkene;
      C) optionally a solvent; and
      D) an acid; and
   II) reacting the mixture.
Embodiment 217. The method of embodiment 216, wherein the cycloalkene or heterocycloalkene is selected from 3,4-dihydropyran, 3,6-dihydropyran, cyclohexene, and combinations thereof.
Embodiment 218. The method of embodiment 216, wherein the solvent is selected from toluene, xylene, heptanes, and combinations thereof.
Embodiment 219. The method of embodiment 216, wherein the acid is selected from organic acid, inorganic acid, and combinations thereof.
Embodiment 220. The method of embodiment 216, wherein the acid is selected from trifluoroacetic acid, sulfuric acid, p-toluenesulfonic acid, methylsulfonic acid, hydrochloric acid, hydrobromic acid, phosphoric acid, and combinations thereof.
Embodiment 221. The method of embodiment 216, wherein the method step of reacting the mixture occurs at a reaction temperature in the range of about 25 °C to about 120 °C.
Embodiment 222. The method of embodiment 221, wherein the method step of reacting the mixture occurs at a reaction temperature in the range of about 60 °C to about 95 °C.

In one aspect, a compound of Formula VI is prepared according to a method represented by Scheme 1. The R groups are as defined anywhere in this disclosure.

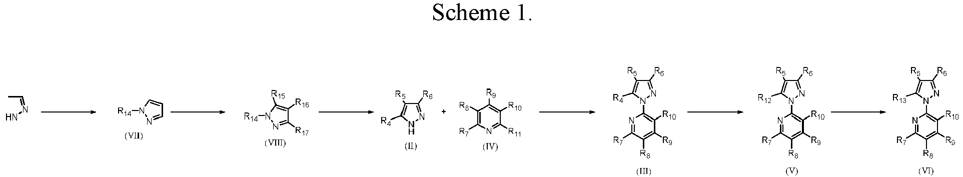

In one aspect, a compound of Formula VI is prepared according to a method represented by Scheme 2. The R groups are as defined anywhere in this disclosure.

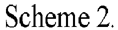

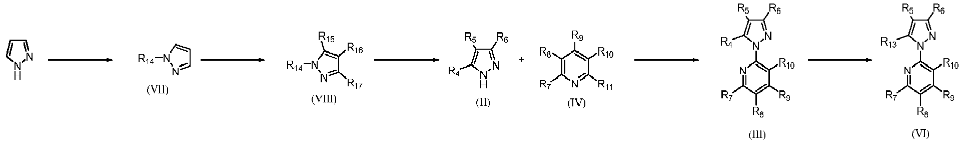

In one aspect, 5-Bromo-2-(3-chloro-pyridin-2-yl)-2H-pyrazole-3-carboxylic acid is prepared according to a method represented by Scheme 3.

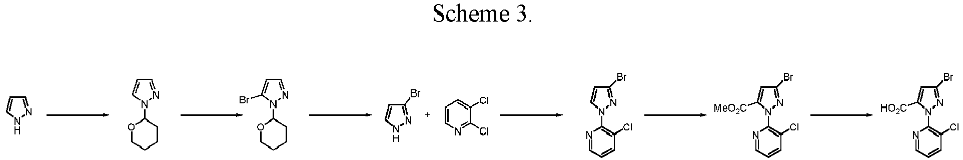

In one aspect, 5-Bromo-2-(3-chloro-pyridin-2-yl)-2H-pyrazole-3-carboxylic acid is prepared according to a method represented by Scheme 4.

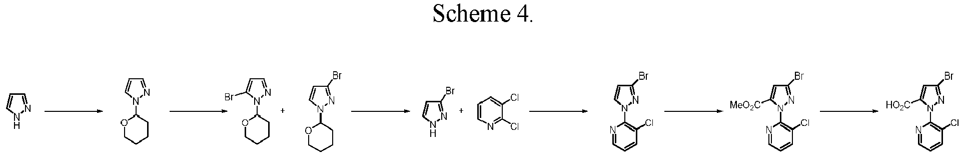

In one aspect, 5-Bromo-2-(3-chloro-pyridin-2-yl)-2H-pyrazole-3-carboxylic acid is prepared according to a method represented by Scheme 5.

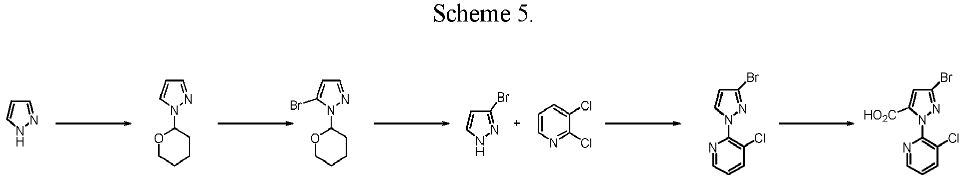

In one aspect, 5-Bromo-2-(3-chloro-pyridin-2-yl)-2H-pyrazole-3-carboxylic acid is prepared according to a method represented by Scheme 6.

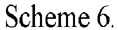

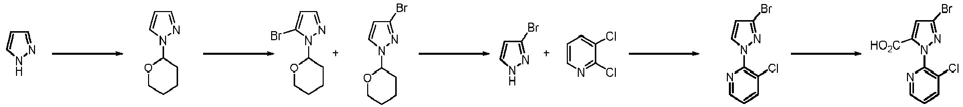

In one aspect, a compound of Formula VII is prepared according to a method represented by Scheme 7. The R groups are as defined anywhere in this disclosure.

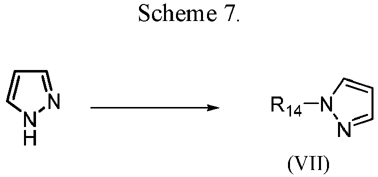

This aspect includes reacting pyrazole with 3,4-dihydropyran (DHP) in the presence of an acid and optionally in an organic solvent. In one embodiment, the organic solvent is selected from toluene, xylene, heptane, and combinations thereof. In another embodiment, the organic solvent is toluene. In one embodiment, the acid is selected from organic acid, inorganic acid, and combinations thereof. In another embodiment, the acid is selected from trifluoroacetic acid, sulfuric acid, p-toluenesulfonic acid, methylsulfonic acid, hydrochloric acid, hydrobromic acid, phosphoric acid, and combinations thereof. In one embodiment, the acid is used in a catalytic amount. In another embodiment, the acid is used in a catalytic amount in the range from about 1 mol% to about 10 mol%. In another embodiment, the acid is a catalytic amount of trifluoroacetic acid or sulfuric acid. In one embodiment, the reaction temperature is in the range from about 25 °C to about 120 °C. In another embodiment, the reaction temperature is in the range from about 60 °C to about 95 °C.

In one aspect, a compound of Formula VIII is prepared according to a method represented by Scheme 8. The R groups are as defined anywhere in this disclosure.

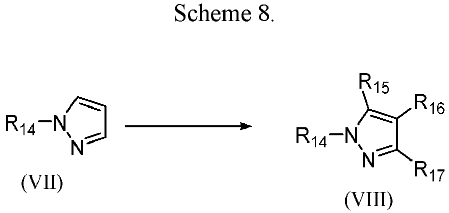

This aspect includes reacting a compound of Formula VII with a base reagent in an organic solvent, followed by a halogenation reagent. In one embodiment, the compound of Formula VII, an organic solvent, and a base reagent are mixed first to form a metalated intermediate, and then the resulting mixture is reacted with a halogenation reagent. In one embodiment, the halogenation reagent is selected from Br₂, N-bromosuccinimide, 1,3-Dibromo-5,5-dimethylhylhydantoin, DBTCE, and combinations thereof. In another embodiment, the halogenation reagent is selected from bromine, N-bromosuccinimide, 1,3-dibromo-5,5-dimethylhylhydantoin, 1,2-dibromotetrachloroethane, tetrabutylammonium tribromide, quaternary ammonium salts of tribromide, 1,2-dibromotrichloroethane, diethyl dibromomalonate, and combinations thereof. In another embodiment, the halogenation reagent is DBTCE. In one embodiment, the solvent is selected from THF, toluene, 1,4-dioxane, 2-Me-THF, and combinations thereof. In another embodiment, the solvent is THF. In one embodiment, the base reagent is selected from *i*Pr₂NMgCl, *i*Pr₂NMgBr, Et₂NMgCl, TMPMgCl, TMPMgCl•LiCl, *i*PriNMgCl•LiCl, *i*Pr₂NMgBr•LiCl, and combinations thereof. In another embodiment, the base reagent is *i*Pr₂NMgCl. In another embodiment, the base reagent is selected from a combination of a Grignard reagent, such as MeMgCl, MeMgBr, iPrMgCl, iPrMgBr, nBuMgCl, nBuMgBr, *t*BuMgCl, and combinations thereof and an amine base, such as *i*Pr₂NH, TMP, Et₂NH, c-Hexyl₂NH, and combinations thereof. In another embodiment, the base reagent is selected from a combination of a Grignard reagent and a catalytic amount of an amine base. In another embodiment, the base reagent is selected from a combination of a Grignard reagent and a catalytic amount of an amine base, wherein the catalytic amount of the amine base in is the range from about 1 mol% to about 90 mol%. In another embodiment, the base reagent is selected from a combination of a Grignard reagent and a catalytic amount of an amine base, wherein the catalytic amount of the amine base in is the range from about 5 mol% to about 50 mol%. In another embodiment, the base reagent is selected from a combination of a Grignard reagent and a catalytic amount of an amine base, wherein the catalytic amount of the amine base in is the range from about 10 mol% to about 20 mol%. In another embodiment, the base reagent is a combination of MeMgCl with a catalytic amount of *i*Pr₂NH. In one embodiment, the reaction temperature is in the range from about 0 °C to about 60 °C. In another embodiment, the reaction temperature is in the range from about 40 °C to about 50 °C. In one embodiment, this aspect produces two distinct compounds of Formula VIII. In another embodiment, this aspect produces two distinct compounds of Formula VIII, wherein the distinct compounds of Formula VIII are isomers. In another embodiment, the two distinct compounds of Formula VIII are 5-bromo-1-(tetrahydro-2H-pyran-2-yl)-1H-pyrazole and 3-bromo-1-(tetrahydro-2H-pyran-2-yl)-1H-pyrazole.

In one aspect, a compound of Formula II is prepared according to a method represented by Scheme 9. The R groups are as defined anywhere in this disclosure.

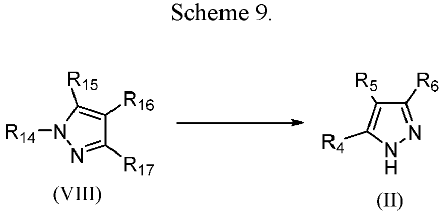

This aspect includes reacting a compound of Formula VIII with an acid in an organic solvent. In one embodiment, the organic solvent is selected from MeOH, EtOH, iPrOH, toluene, 1,4-dioxane, THF, and combinations thereof. In another embodiment, the solvent is MeOH. In one embodiment, the acid is selected from hydrochloric acid, sulfuric acid, phosphoric acid, methylsulfonic acid, trifluoroacetic acid, hydrobromic acid, and combinations thereof. In another embodiment, the acid is MeSOsH. In one embodiment, the reaction temperature is in the range from about 20 °C to 150 °C. In another embodiment, the reaction temperature is in the range from about 20 °C to 50 °C. In one embodiment, this aspect includes reacting at least two distinct compounds of Formula VIII. In another embodiment, this aspect includes reacting 5-bromo-1-(tetrahydro-2H-pyran-2-yl)-1H-pyrazole and 3-bromo-1-(tetrahydro-2H-pyran-2-yl)-1H-pyrazole.

In one aspect, a compound of Formula III is prepared according to a method represented by Scheme 10. The R groups are as defined anywhere in this disclosure.

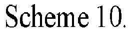

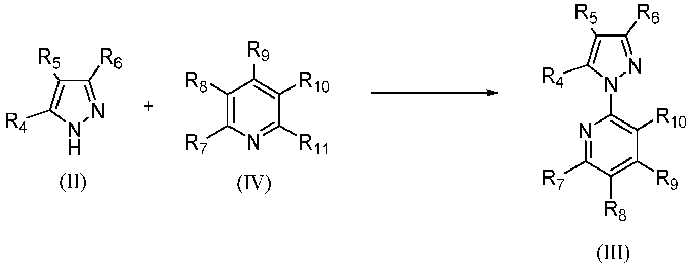

This aspect includes mixing a compound of Formula II with a compound of Formula IV in a solvent in the presence of an inorganic base and optionally an additive. In one embodiment, the inorganic base is selected from powder sodium hydroxide, powder potassium hydroxide, potassium carbonate, potassium phosphate powder sodium methoxide, powder potassium t-butoxide, and combinations thereof. In another embodiment, the inorganic base is powdered potassium phosphate. In one embodiment, the solvent is selected from toluene, N,N-Dimethylformamide (DMF), N,N-Dimethylacetamide (DMAc), N-methyl-2-pyrrolidone (NMP), acetonitrile, and combinations thereof. In another embodiment, the solvent is acetonitrile. In one embodiment, the reaction temperature is in the range from about 100 °C to about 200 °C. In another embodiment, the reaction temperature is in the range from about 100 °C to about 180 °C. In another embodiment, the reaction temperature is in the range from about 130 °C to about 180 °C. In another embodiment, the temperature is in the range from about 145 °C to about 160 °C. In another embodiment, the temperature is in the range from about 135 °C to about 145 °C.

In one aspect, a compound of Formula VI is prepared according to a method represented by Scheme 11. The R groups are as defined anywhere in this disclosure.

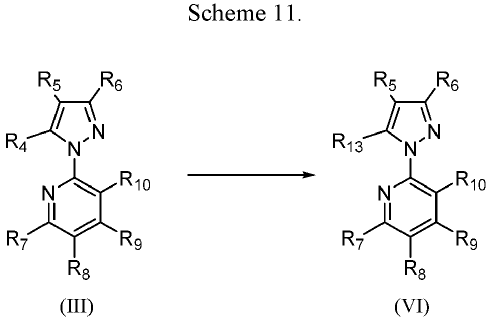

This aspect includes mixing a compound of Formula III with CO₂ in a solvent in the presence of a base reagent and optionally an additive. In one embodiment, the compound of Formula III, a solvent, a base reagent, and optionally an additive are mixed first to form a metalated intermediate, and then the resulting mixture is reacted with CO₂. In one embodiment, the compound of Formula III, a solvent, a base reagent, optionally an additive, and CO₂ are concurrently mixed and subsequently reacted. In one embodiment, the base reagent is selected from *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof. In another embodiment, the base reagent is *i*Pr₂NMgCl. In one embodiment, the base reagent is selected from a combination of a Grignard reagent and an amine base. In another embodiment, the base reagent is selected from a combination of a Grignard reagent and a catalytic amount of an amine base. In another embodiment, the base reagent is selected from a combination of a Grignard reagent selected from MeMgCl, MeMgBr, iPrMgCl, iPrMgBr, nBuMgCl, nBuMgBr, tBuMgCl, and combinations thereof, and a catalytic amount of an amine base selected from *i*Pr₂NH, TMP, Et₂NH, hexamethyldisilazane (HMDS), c-Hexyl₂NH, and combinations thereof. In other embodiment, the base reagent is a combination of MeMgCl and *i*Pr₂NH. In one embodiment, the solvent is selected from THF, toluene, 1,4-dioxane, Me-THF, and combinations thereof. In another embodiment, the solvent is THF. In another embodiment, the solvent is THF. In one embodiment, the reaction temperature is in the range from about 0 °C to about 60 °C. In another embodiment, the temperature is in the range from about 0 °C to about 30 °C. In another embodiment, the temperature is in the range from about 10 °C to about 20 °C. In one embodiment, the additive is selected from LiCl, TMEDA, and combinations thereof.

In one aspect, a compound of Formula V is prepared according to a method represented by Scheme 12. The R groups are as defined anywhere in this disclosure.

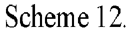

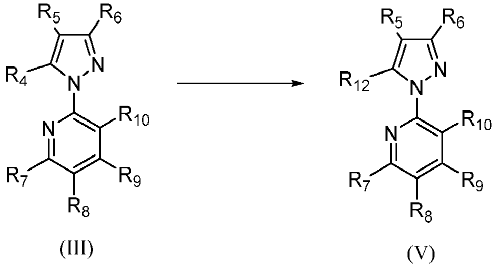

This aspect includes mixing a compound of Formula III with dimethyl carbonate (DMC) in a solvent in the presence of a base reagent and optionally an additive. In one embodiment, the compound of Formula III, a solvent, a base reagent, and optionally an additive are mixed first to form a metalated intermediate, and then the resulting mixture is reacted with dimethyl carbonate. In one embodiment, the compound of Formula III, a solvent, a base reagent, optionally an additive, and dimethyl carbonate are concurrently mixed and subsequently reacted.

In one embodiment, the base reagent is selected from *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof. In another embodiment, the base reagent is *i*Pr₂NMgCl. In one embodiment, the base reagent is selected from a combination of a Grignard reagent and an amine base. In another embodiment, the base reagent is selected from a combination of a Grignard reagent and a catalytic amount of an amine base. In another embodiment, the base reagent is selected from a combination of a Grignard reagent selected from MeMgCl, MeMgBr, iPrMgCl, iPrMgBr, nBuMgCl, nBuMgBr, tBuMgCl, and combinations thereof, and a catalytic amount of an amine base selected from *i*Pr₂NH, TMP, Et₂NH, hexamethyldisilazane (HMDS), c-Hexyl₂NH, and combinations thereof. In other embodiment, the base reagent is a combination of MeMgCl and *i*Pr₂NH. In one embodiment, the solvent is selected from THF, toluene, 1,4-dioxane, Me-THF, and combinations thereof. In another embodiment, the solvent is THF. In one embodiment, the reaction temperature is in the range from about 0 °C to about 50 °C. In another embodiment, the temperature is in the range from about 10 °C to about 25 °C. In one embodiment, the additive is selected from LiCl, TMEDA, and combinations thereof.

In one aspect, a compound of Formula VI is prepared according to a method represented by Scheme 13. The R groups are as defined anywhere in this disclosure.

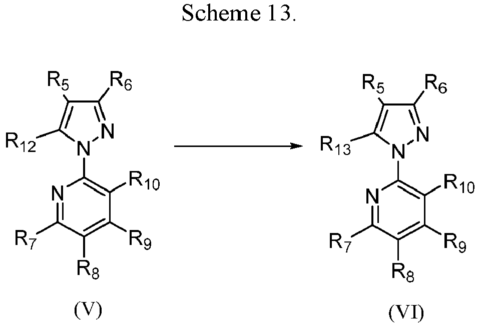

This aspect includes reacting a compound of Formula V with an aqueous metal hydroxide solution. In one embodiment, the metal hydroxide is selected from alkali hydroxide, alkaline earth metal hydroxide, and combinations thereof. In one embodiment, the alkali hydroxide is selected from lithium hydroxide, sodium hydroxide, potassium hydroxide, and combinations. In one embodiment, the alkaline earth metal hydroxide is selected from calcium hydroxide, barium hydroxide, and combinations thereof. In another embodiment, the metal hydroxide is sodium hydroxide or potassium hydroxide. In one embodiment, the reaction temperature is in the range from about 0 °C to about 90 °C. In another embodiment, the reaction temperature is in the range from about 30 °C to about 60 °C. In another embodiment, the reaction temperature is in the range from about 60 °C to about 80 °C. In another embodiment, the reaction temperature is in the range from about 40 °C to about 70 °C.

### EXAMPLES

Without further elaboration, it is believed that one skilled in the art using the preceding description can utilize the present invention to its fullest extent. The following Examples are, therefore, to be construed as merely illustrative, and not limiting of the disclosure in any way whatsoever. The starting material for the following Examples may not have necessarily been prepared by a particular preparative run whose procedure is described in other Examples. It also is understood that any numerical range recited herein includes all values from the lower value to the upper value. For example, if a range is stated as 10-50, it is intended that values such as 12-30, 20-40, or 30-50, etc., are expressly enumerated in this specification. These are only examples of what is specifically intended, and all possible combinations of numerical values between and including the lowest value and the highest value enumerated are to be considered to be expressly stated in this application.

### Example 1. Protecting reaction.

0.2 mL of trifluoracetic acid (TFA) was added to a mixture of 23.2 g pyrazole and 31.5 g of DHP over 1 minute. The resulting mixture was self-heated to over 130 °C initially. The reaction was held at 130 °C for 1 hour. The crude light brown oil was purified by distillation under reduced pressure. 47 grams of clear liquid was collected at 90 °C.

### Example 2. Protecting reaction.

1.46 grams of p-toluenesulfonic acid monohydrate was added to a mixture of 104.7 g pyrazole and 350 mL of toluene, and subsequently heated to 87 °C. Next, 145 g of 3,4-dihydro-2*H*-pyran was added over 30 minutes. The resulting mixture was stirred between 90 and 95 °C for 2.5 hours. After cooling to 30 °C, the solution was washed with 300 mL of saturated sodium bicarbonate aqueous solution and concentrated to a yellow oil (242 g).

### Example 3. Halogenation.

5 mL of *i*Pr₂NH and 120 mL of 1.3 M *i*PrMgCl/LiCl in THF was added to a solution of 19.4 g of 1-(tetrahydro-2H-pyran-2-yl)-1H-pyrazole in 225 mL THF over 1 hour at room temperature. The temperature was maintained at a temperature from 19 °C to 27 °C during addition. The resulting mixture was stirred overnight at room temperature. After cooling to 5 °C, a solution of 1,2-dibromotetrachloroethane (51 g in 100 mL THF) was added over 30 min, during which the temperature was maintained below 10 °C. After warming to room temperature, saturated NH₄Cl (aq) was added. The organic layer was separated and concentrated to afford a dark oil comprising 5-bromo-1-(tetrahydro-2H-pyran-2-yl)-1H-pyrazole and 3-bromo-1-(tetrahydro-2H-pyran-2-yl)-1H-pyrazole.

### Example 4. Halogenation.

2.0 mL of *i*Pr₂NH and 50 mL of 3 M MeMgCl in THF was added to a solution of 18.3 g of 1-(tetrahydro-2H-pyran-2-yl)-1H-pyrazole in 210 mL THF over 10 minutes at room temperature. The resulting mixture was stirred at 50 °C for 2 hours. After cooling to 10 °C, a solution of 1,2-dibromotetrachloroethane (43 g in 70 mL THF) was added over 20 min. After warming to room temperature, 300 mL of water and 20 mL of 2 N HCl was added. The organic layer was separated and concentrated to afford a brown oil comprising 5-bromo-1-(tetrahydro-2H-pyran-2-yl)-1H-pyrazole and 3-bromo-1-(tetrahydro-2H-pyran-2-yl)-1H-pyrazole.

### Example 5. Halogenation.

6.2 mL of *i*Pr₂NH was added to a solution of 71 g of 1-(tetrahydro-2H-pyran-2-yl)-1H-pyrazole in 800 mL THF at room temperature. 200 mL of 3 M MeMgCl in THF was then added over 30 minutes. The resulting mixture was stirred at room temperature for 20 hours. After cooling to 6 °C, 148 g of 1,2-dibromotetrachloroethane solid was added portion-wise over 30 minutes, during which the temperature was maintained below 15 °C. Next, the mixture was stirred at 15 °C for 1 hour, and then a solution of 60 g NaOAc in 300 mL water was added. The organic layer was separated and concentrated to 122.7 g of dark brown oil comprising 5-bromo-1-(tetrahydro-2H-pyran-2-yl)-1H-pyrazole as a major component and 3-bromo-1-(tetrahydro-2H-pyran-2-yl)-1H-pyrazole as a minor component. After standing at room temperature for 3 days, 5-bromo-1-(tetrahydro-2H-pyran-2-yl)-1H-pyrazole was isomerized to 3-bromo-1-(tetrahydro-2H-pyran-2-yl)-1H-pyrazole. 85.2 g of 3-bromo-1-(tetrahydro-2H-pyran-2-yl)-1H-pyrazole was obtained after purification by column chromatography.

### Example 6. Removal of protecting group.

To 12 g of crude oil (5-bromo-1-(tetrahydro-2H-pyran-2-yl)-1H-pyrazole containing 3-bromo-1-(tetrahydro-2H-pyran-2-yl)-1H-pyrazole), 50 mL of MeOH and 50 mL of 3 M HCl in MeOh was added. The resulting mixture was stirred at room temperature for four hours, and then concentrated on rotavapor. The residue was diluted with 100 mL of EtOAc, washed with saturated NaHCOs, and concentrated to afford 11 g of crude 3-bromo-1H-pyrazole.

### Example 7. Removal of protecting group.

To 2 g of crude oil (5-bromo-1-(tetrahydro-2H-pyran-2-yl)-1H-pyrazole), 20 mL of MeOH and 1 mL of MeSO₃H was added. The resulting mixture was stirred at room temperature for two hours, and then concentrated on rotavapor. The residue was diluted with 20 mL of EtOAc, washed with saturated NaHCO₃, and concentrated to afford 1.6 g of crude 3-bromo-1H-pyrazole.

### Example 8. Removal of protecting group.

To a solution of 109.7 g of crude oil (5-bromo-1-(tetrahydro-2H-pyran-2-yl)-1H-pyrazole containing 3-bromo-1-(tetrahydro-2H-pyran-2-yl)-1H-pyrazole) in 8000 mL MeOH, 96.2 g of MeSO₃H was added. The resulting mixture was stirred at room temperature for two hours, and then concentrated to a dark oil. With agitation, 3 N NaOH was added until the pH was about 8. Next, NaCl solid was added until saturation and then extracted with 300 mL of EtOAc. The organic layer was separated and concentrated to afford 64.6 g of crude 3-bromo-1H-pyrazole as a brown oil.

### Example 9. Coupling reaction.

151 g of 2,3-dichloropyridine (151 g), 135.9 g of 3-bromo-1H-pyrazole, 210 g of powdered K₃PO₄, and 700 mL of CH₃CN were charged into an autoclave. The mixture was sealed and stirred at 140 °C for 8 hours. After cooling to room temperature, 400 mL of water was added and the organic layer was separated. The solvent was removed by distillation to afford 220 g of 2-(3-bromo-1H-pyrazol-1-yl)-3-chloropyridine as a solid.

### Example 10. Reaction in the presence of a Grignard reagent.

9.3 mL of a 1.0 M TMPMgCl•LiCl solution in THF was added to a solution of 2.04 g 2-(3-bromo-1H-pyrazol-1-yl)-3-chloropyridine in 28 mL of THF at 8 °C over 30 minutes. The resulting mixture was stirred at 10 °C for 2 hours. Gaseous CO₂ was introduced into the solution for 5 minutes, and then 100 mL water was added, and the pH was adjusted to about 9 by adding 1N NaOH aqueous solution. The resulting solution was washed with 50 mL toluene and the aqueous layer was acidified by 2N HCl (aq) to a pH of about 3, and subsequently extracted with 50 mL EtOAc. The organic phase was separated and concentrated to provide 1.9 g of 5-bromo-2-(3-chloro-pyridin-2-yl)-2H-pyrazole-3-carboxylic acid as an off-white solid.

### Example 11. Reaction in the presence of a Grignard reagent.

22 mL of a 0.4 M *i*Pr₂NMgCl•LiCl solution in THF (freshly prepared from *i*Pr₂NH and zPrMgCl.LiCl in THF overnight at room temperature) was added to a solution of 2.0 g 2-(3-bromo-1H-pyrazol-1-yl)-3-chloropyridine in 20 mL of THF at 2 °C over 10 minutes. The resulting mixture was stirred at 5 °C for 3 hours. Gaseous CO₂ was introduced into the solution for 5 minutes, and then 50 mL water was added, and the pH was adjusted to about 9 by adding 1N NaOH aqueous solution. An extraction with 30 mL toluene afforded 1.23 g of recovered 2-(3-bromo-1H-pyrazol-1-yl)-3-chloropyridine. The aqueous layer was acidified by 2N HCl (aq) to a pH of about 2, and subsequently extracted with 50 mL EtOAc to afford 0.82 g of 5-bromo-2-(3-chloro-pyridin-2-yl)-2H-pyrazole-3-carboxylic acid as a beige solid.

### Example 12. Reaction in the presence of a Grignard reagent.

4.0 mL of a 3 M MeMgCl solution in THF was added to a solution of 2.0 g 2-(3-bromo-1H-pyrazol-1-yl)-3-chloropyridine in 20 mL of toluene, 0.39 mL HMDS, and 1.3 mL TMEDA at 7.5 °C over 10 minutes. The resulting mixture was stirred at 7 °C overnight (17 hours). Gaseous CO₂ was introduced into the solution for 5 minutes, and then 50 mL water was added, and the pH was adjusted to about 9. The organic phase was separated and concentrated to afford 0.71 g of recovered 2-(3-bromo-1H-pyrazol-1-yl)-3-chloropyridine as a yellow solid. The aqueous layer was acidified by 2N HCl (aq) to a pH of about 2, and subsequently extracted with 20 mL EtOAc to afford 0.77 g of 5-bromo-2-(3-chloro-pyridin-2-yl)-2H-pyrazole-3-carboxylic acid.

### Example 13. Reaction in the presence of a Grignard reagent.

A mixture of *i*Pr₂NMgCl in THF (prepared from 8 mL *i*Pr₂NH and 18 mL of a 3 M MeMgCl solution in THF) was added to a solution of 10.0 g 2-(3-bromo-1H-pyrazol-1-yl)-3-chloropyridine in 40 mL of toluene and 6.0 mL TMEDA at 0 °C over 10 minutes. The resulting mixture was stirred at 0 °C for 3 hours. Gaseous CO₂ was introduced into the solution for 10 minutes, and then 100 mL water was added, and the pH was adjusted to about 9. The organic phase was separated and concentrated to afford 6.6 g of recovered 2-(3-bromo-1H-pyrazol-1-yl)-3-chloropyridine. The aqueous layer was acidified by 2N HCl (aq) to a pH of about 2, and the slurry was filtered and dried to afford 3.2 g of 5-bromo-2-(3-chloro-pyridin-2-yl)-2H-pyrazole-3-carboxylic acid as a yellow solid.

### Example 14. Reaction in the presence of a Grignard reagent.

A mixture of *i*Pr₂NMgCl in THF (prepared from 32 g *i*Pr₂NH and 98 mL of a 3 M MeMgCl solution in THF) was added to a solution of 60.0 g 2-(3-bromo-1H-pyrazol-1-yl)-3-chloropyridine and 11 g LiCl in 300 mL THF and 50 mL toluene at 5 °C over 45 minutes. The resulting mixture was stirred at 8 °C to 15 °C for 6 hours. Gaseous CO₂ was introduced into the solution for 15 minutes, and then 100 mL water was added, and the pH was adjusted to about 1 with 2 N HCl. The organic phase was separated and concentrated by rotary evaporation. The residue was treated with 200 mL 2N NaOH and extracted with 100 mL toluene to afford 17.2 g of recovered 2-(3-bromo-1H-pyrazol-1-yl)-3-chloropyridine. The aqueous layer was acidified by 2N HCl (aq) to a pH of about 1, and the slurry was filtered and dried to afford 46 g of 5-bromo-2-(3-chloro-pyridin-2-yl)-2H-pyrazole-3-carboxylic acid.

### Example 15. Reaction in the presence of a Grignard reagent and hydrolysis.

A mixture of *i*Pr₂NMgCl in THF (prepared from mixing 10.9 g *i*Pr₂NH and 35 mL of a 3 M MeMgCl solution in THF in 50 mL THF at 50 °C for 3 hours) was added to a solution of 20.1 g 2-(3-bromo-1H-pyrazol-1-yl)-3-chloropyridine and 3.6 g LiCl in 100 mL THF at 5 °C over 30 minutes. The resulting mixture was stirred at 10 °C for 3 hours, and then 60 mL dimethyl carbonate was added. The resulting mixture was stirred at 45 °C for 3 hours. 200 mL 1 N HCl was added, and the organic phase was separated and concentrated to 27 g crude methyl 3-bromo-1-(3-chloropyridin-2-yl)-1H-pyrazole-5-carboxylate. 80 mL toluene and 200 mL 1 N NaOH was added and the mixture was stirred at 60 °C for 3 hours. The aqueous phase was separated and acidified with concentrated HCL to a pH of about 1, and then the solid was filtered and dried to afford 16.1 g of 5-bromo-2-(3-chloro-pyridin-2-yl)-2H-pyrazole-3-carboxylic acid as a solid.

This written description uses examples to illustrate the present disclosure, including the best mode, and also to enable any person skilled in the art to practice the disclosure, including making and using any devices or systems and performing any incorporated methods. The patentable scope of the disclosure is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

Further aspects of the invention (Aspects A1-A19) are as follows:
A1. A method of preparing a compound of Formula VI, wherein 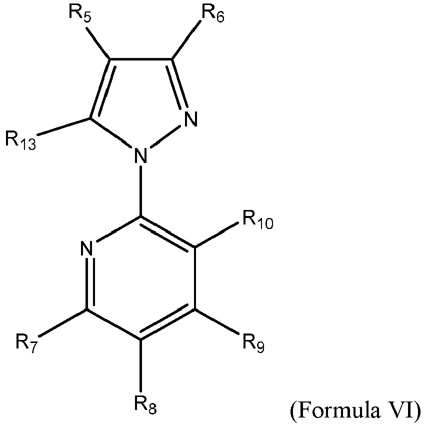
   each of R₅ - R₁₀ is independently selected from hydrogen and halogen; and
   R₁₃ is an organic acid, the method comprising
      I) forming a mixture comprising
         A) a compound of Formula V, wherein 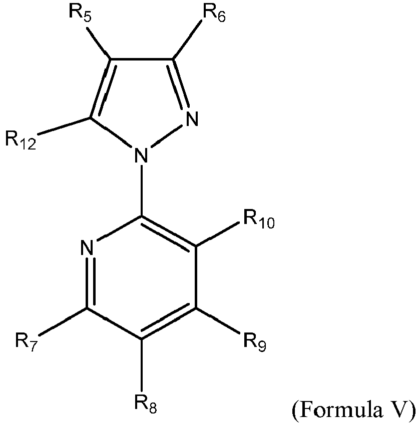
            each of R₅ - R₁₀ is independently selected from hydrogen and halogen;
            R₁₂ is selected from ether, ester, and nitrile; and
            wherein the compound of Formula V is prepared according to a method comprising
               i) forming a mixture comprising
                  a) a compound of Formula III, wherein 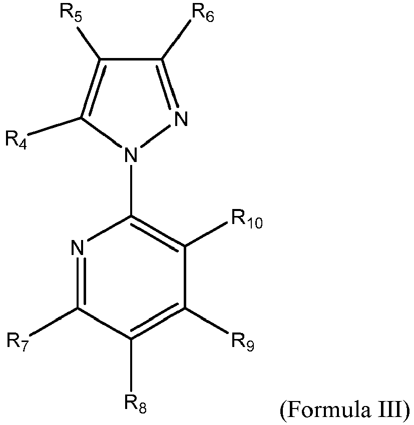
                     R₄ is hydrogen;
                     each of R₅ - R₁₀ is independently selected from hydrogen and halogen; and
                     wherein the compound of Formula III is prepared according to a method comprising
                        IA) forming a mixture comprising
                           AA) a compound of Formula II, wherein 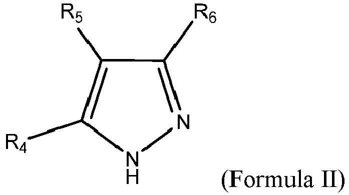
                              each of R₄, R₅, and R₆ is independently selected from hydrogen and halogen;
                              wherein at least one of R₄, Rs, and R₆ is hydrogen; and
                              wherein the compound of Formula II is prepared according to a method comprising
                                 ia) forming a mixture comprising
                                    aa) a compound of Formula VIII, wherein 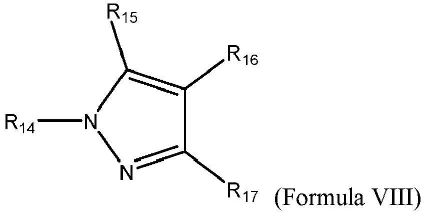
                                       R₁₄ is selected from substituted or unsubstituted carbocycle and substituted or unsubstituted heterocycle;
                                       each of R₁₅, R₁₆, and R₁₇ is independently selected from hydrogen and halogen; and
                                       wherein at least one of R₁₅, R₁₆, and R₁₇ is a halogen;
                                    bb) a solvent; and
                                    cc) an acid; and
                                 iia) reacting the mixture;
                           BB) a compound of Formula IV, wherein 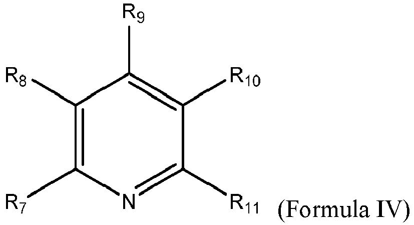 each of R₇- R₁₁ is independently selected from hydrogen and halogen;
                           CC) a solvent;
                           DD) an inorganic base; and
                           EE) optionally an additive; and
                        IIA) reacting the mixture;
                  b) a solvent;
                  c) a base comprising
                     a compound comprising a metal; and
                     optionally a first amine base;
                  d) optionally an additive; and
                  e) optionally a second amine base; and
            ii) reacting the mixture with an organic compound; and
         B) a metal hydroxide; and
      II) reacting the mixture.
A2. The method of Aspect A1, wherein the metal hydroxide is selected from alkali hydroxide, alkaline earth metal hydroxide, and combinations thereof.
A3. The method of Aspect A1, wherein the method step II) of reacting the mixture occurs at a reaction temperature in the range of about 0 °C to about 90 °C.
A4. The method of Aspect A1, wherein the compound comprising a metal is a Grignard reagent.
A5. The method of Aspect A1, wherein the first amine base and the second amine base are each independently selected from *i*Pr₂NH, TMP, hexamethyldisilazane, Et₂NH, c-hexyl₂NH, and combinations thereof.
A6. The method of Aspect A1, wherein the solvent b) is selected from THF, toluene, 1,4-dioxane, Me-THF, and combinations thereof.
A7. The method of Aspect A1, wherein the method step ii) of reacting the mixture occurs at a reaction temperature in the range of about 0 °C to about 50 °C.
A8. The method of Aspect A1, wherein the compound of Formula VIII is prepared according to a method comprising
   I) forming a mixture comprising
      A) a compound of Formula VII, wherein 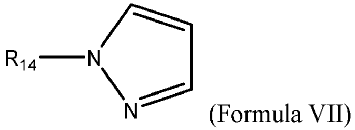 R₁₄ is selected from substituted or unsubstituted carbocycle and substituted or unsubstituted heterocycle;
      B) a solvent;
      C) a base comprising
         a compound comprising a metal; and
         optionally a first amine base; and
      D) optionally a second amine base; and
   II) reacting the mixture with a halogenation reagent.
A9. The method of Aspect A8, wherein the method produces a compound selected from 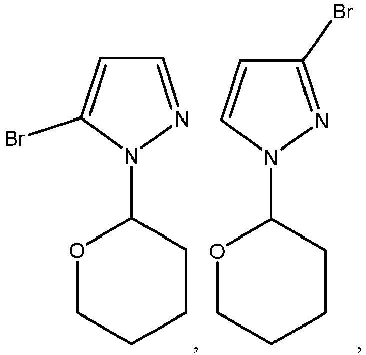 and combinations thereof.
A10. A method of preparing a compound of Formula VI, wherein 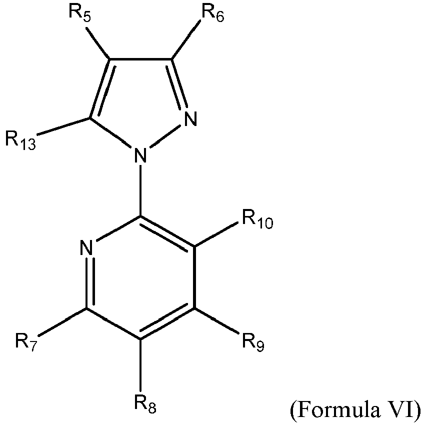
   each of R₅ - R₁₀ is independently selected from hydrogen and halogen; and
   R₁₃ is an organic acid, the method comprising
      I) forming a mixture comprising
         A) a compound of Formula III, wherein 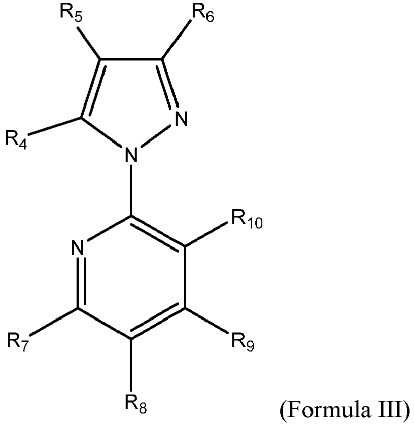
            R₄ is hydrogen;
            each of R₅ - R₁₀ is independently selected from hydrogen and halogen; and
            wherein the compound of Formula III is prepared according to a method comprising
               i) forming a mixture comprising
                  a) a compound of Formula II, wherein 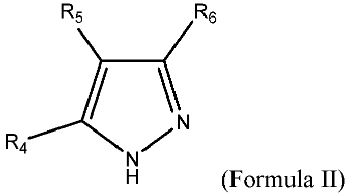
                     each of R₄, R₅, and R₆ is independently selected from hydrogen and halogen;
                     wherein at least one of R₄, Rs, and R₆ is hydrogen; and
                     wherein the compound of Formula II is prepared according to a method comprising
                        IA) forming a mixture comprising
                           AA) a compound of Formula VIII, wherein 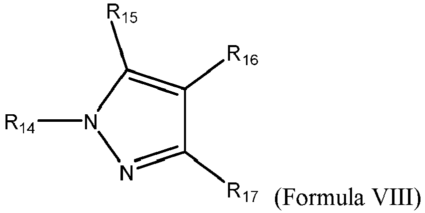
                              R₁₄ is selected from substituted or unsubstituted carbocycle and substituted or unsubstituted heterocycle;
                              each of R₁₅, R₁₆, and R₁₇ is independently selected from hydrogen and halogen; and
                              wherein at least one of R₁₅, R₁₆, and R₁₇ is a halogen;
                           BB) a solvent; and
                           CC) an acid; and
                        IIa) reacting the mixture;
                        b) a compound of Formula IV, wherein 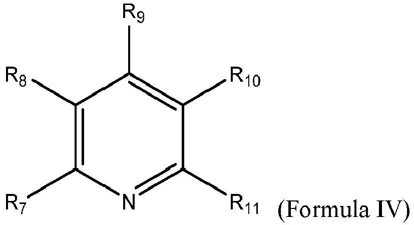 each of R₇ - R₁₁ is independently selected from hydrogen and halogen;
                        c) a solvent;
                        d) an inorganic base; and
                        e) optionally an additive; and
               ii) reacting the mixture;
         B) a solvent;
         C) a base comprising
            a compound comprising a metal; and
            optionally a first amine base;
         D) optionally an additive; and
         E) optionally a second amine base; and
      II) reacting the mixture with a carbonyl-containing compound.
A11. The method of Aspect A10, wherein the compound comprising a metal is a Grignard reagent.
A12. A method of preparing a compound of Formula II, wherein 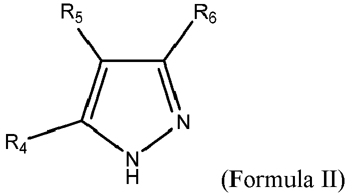
   each of R₄, R₅, and R₆ is independently selected from hydrogen and halogen; and
   wherein at least one of R₄, Rs, and R₆ is hydrogen, the method comprising
      I) forming a mixture comprising
         A) a compound of Formula VIII, wherein 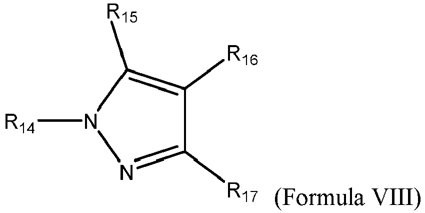
            R₁₄ is selected from substituted or unsubstituted carbocycle and substituted or unsubstituted heterocycle;
            each of R₁₅, R₁₆, and R₁₇ is independently selected from hydrogen and halogen; and
            wherein at least one of R₁₅, R₁₆, and R₁₇ is a halogen;
         B) a solvent; and
         C) an acid; and
      II) reacting the mixture.
A13. The method of Aspect A12, wherein the solvent is selected from methanol, ethanol, isopropanol, toluene, 1,4-dioxane, tetrahydrofuran, and combinations thereof.
A14. A method of preparing a compound of Formula VII, wherein 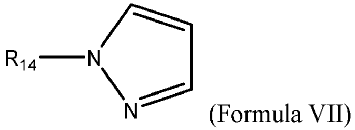 R₁₄ is selected from substituted or unsubstituted carbocycle and substituted or unsubstituted heterocycle, the method comprising
   I) forming a mixture comprising
      A) pyrazole or a pyrazole derivative;
      B) a cycloalkene or heterocycloalkene;
      C) optionally a solvent; and
      D) an acid; and
   II) reacting the mixture.
A15. A method of preparing a compound of Formula VIII, wherein 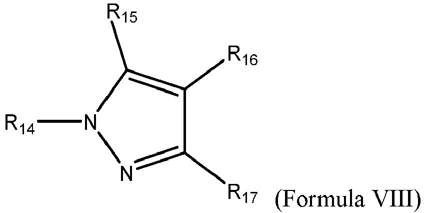
   R₁₄ is selected from substituted or unsubstituted carbocycle and substituted or unsubstituted heterocycle;
   each of R₁₅, R₁₆, and R₁₇ is independently selected from hydrogen and halogen; and
   wherein at least one of R₁₅, R₁₆, and R₁₇ is a halogen, the method comprising
      I) forming a mixture comprising
         A) the compound of Formula VII, wherein 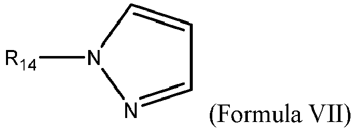 R₁₄ is selected from substituted or unsubstituted carbocycle and substituted or unsubstituted heterocycle;
         B) a solvent;
         C) a base comprising
            a compound comprising a metal; and
            optionally a first amine base; and
         D) optionally a second amine base; and
      II) reacting the mixture with a halogenation reagent.
A16. The method of Aspect A15, wherein the compound comprising a metal is a Grignard reagent.
A17. A method of preparing a compound of Formula V, wherein 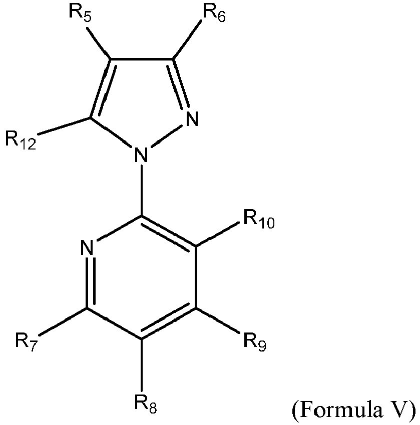
   each of R₅ - R₁₀ is independently selected from hydrogen and halogen; and
   R₁₂ is selected from ether, ester, and nitrile, the method comprising
      i) forming a mixture comprising
         a) a compound of Formula III, wherein 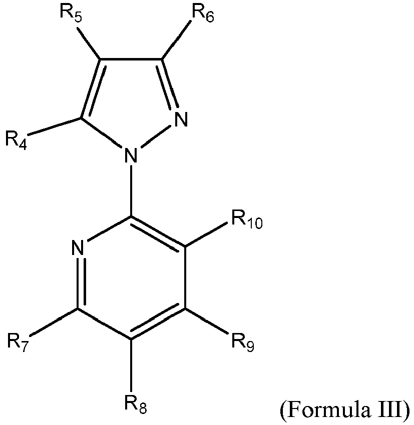
            R₄ is hydrogen;
            each of R₅ - R₁₀ is independently selected from hydrogen and halogen; and
            wherein the compound of Formula III is prepared according to a method comprising
               IA) forming a mixture comprising
                  AA) a compound of Formula II, wherein 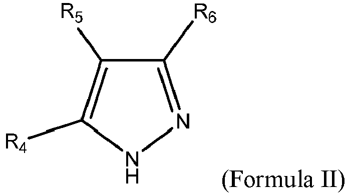
                     each of R₄, R₅, and R₆ is independently selected from hydrogen and halogen;
                     wherein at least one of R₄, Rs, and R₆ is hydrogen; and
                     wherein the compound of Formula II is prepared according to a method comprising
                        ia) forming a mixture comprising
                           aa) a compound of Formula VIII, wherein 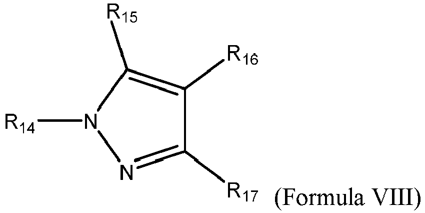
                              R₁₄ is selected from substituted or unsubstituted carbocycle and substituted or unsubstituted heterocycle;
                              each of R₁₅, R₁₆, and R₁₇ is independently selected from hydrogen and halogen; and
                              wherein at least one of R₁₅, R₁₆, and R₁₇ is a halogen;
                           bb) a solvent; and
                           cc) an acid; and
                        iia) reacting the mixture;
                  BB) a compound of Formula IV, wherein 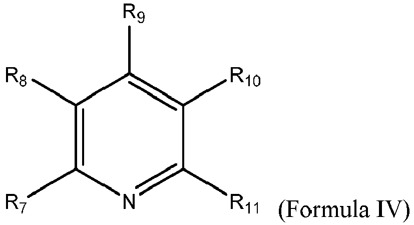 each of R₇ - R₁₁ is independently selected from hydrogen and halogen;
                  CC) a solvent;
                  DD) an inorganic base; and
                  EE) optionally an additive; and
               IIA) reacting the mixture;
         b) a solvent;
         c) a base comprising
            a compound comprising a metal; and
            optionally a first amine base;
         d) optionally an additive; and
         e) optionally a second amine base; and
      ii) reacting the mixture with an organic compound.
A18. The method of Aspect A17, wherein the compound comprising a metal is a Grignard reagent.
A19. A method of preparing a compound of Formula III, wherein 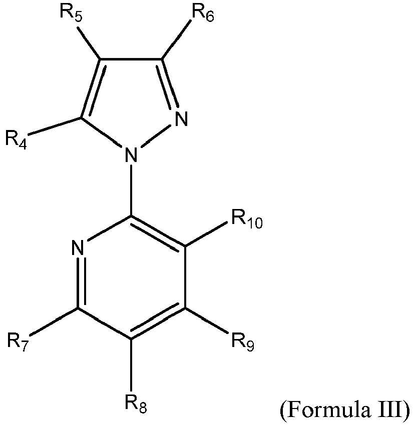
   R₄ is hydrogen; and
   each of R₅ - R₁₀ is independently selected from hydrogen and halogen, the method comprising
      i) forming a mixture comprising
         a) a compound of Formula II, wherein 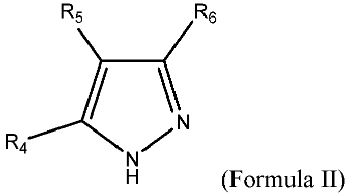
            each of R₄, R₅, and R₆ is independently selected from hydrogen and halogen;
            wherein at least one of R₄, R₅, and R₆ is hydrogen; and
            wherein the compound of Formula II is prepared according to a method comprising
               IA) forming a mixture comprising
                  AA) a compound of Formula VIII, wherein 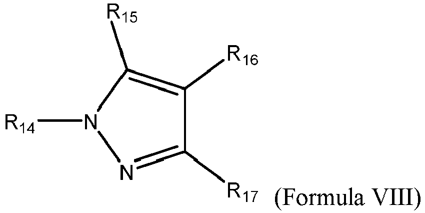
                     R₁₄ is selected from substituted or unsubstituted carbocycle and substituted or unsubstituted heterocycle;
                     each of R₁₅, R₁₆, and R₁₇ is independently selected from hydrogen and halogen; and
                     wherein at least one of R₁₅, R₁₆, and R₁₇ is a halogen;
                  BB) a solvent; and
                  CC) an acid; and
               IIa) reacting the mixture;
         b) a compound of Formula IV, wherein 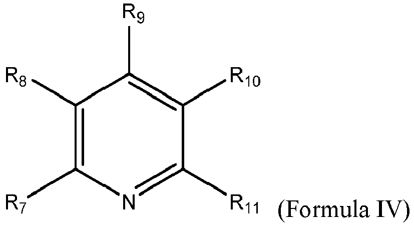 each of R₇ - R₁₁ is independently selected from hydrogen and halogen;
         c) a solvent;
         d) an inorganic base; and
         e) optionally an additive; and
      ii) reacting the mixture.

## Claims

1. A method of preparing a compound of Formula VI, wherein 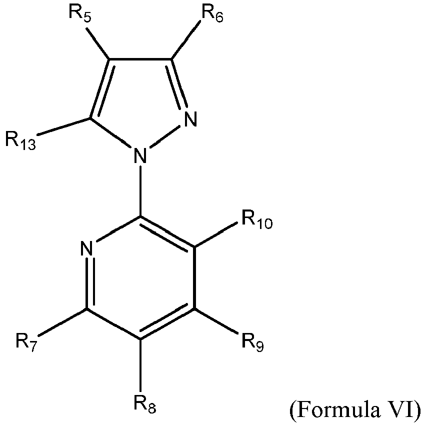
each of R₅ - R₁₀ is independently selected from hydrogen and halogen; and
R₁₃ is an organic acid, the method comprising
I) forming a mixture comprising
A) a compound of Formula V, wherein 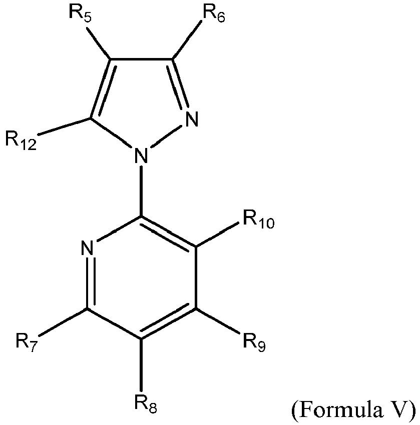
each of R₅ - R₁₀ is independently selected from hydrogen and halogen;
R₁₂ is selected from ether, ester, and nitrile; and
wherein the compound of Formula V is prepared according to a method comprising
i) forming a mixture comprising
a) a compound of Formula III, wherein 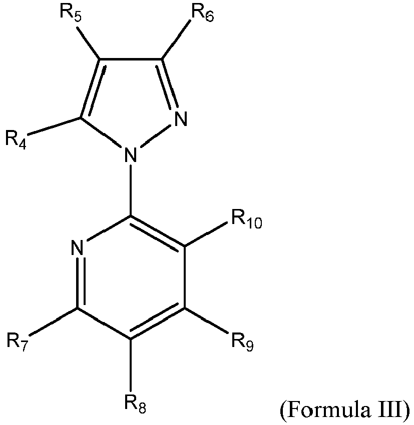
R₄ is hydrogen;
each of R₅ - R₁₀ is independently selected from hydrogen and halogen; and
wherein the compound of Formula III is prepared according to a method comprising
IA) forming a mixture comprising
AA) a compound of Formula II, wherein 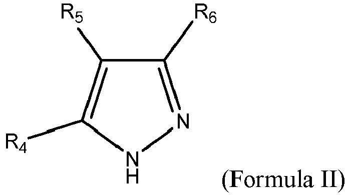
each of R₄, R₅, and R₆ is independently selected from hydrogen and halogen;
wherein at least one of R₄, Rs, and R₆ is hydrogen; and
wherein the compound of Formula II is prepared according to a method comprising
ia) forming a mixture comprising
aa) a compound of Formula VIII, wherein 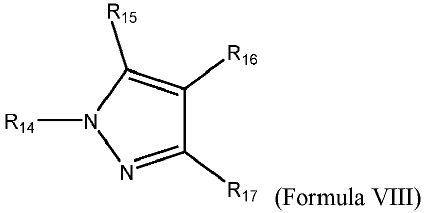
R₁₄ is selected from substituted or unsubstituted carbocycle and substituted or unsubstituted heterocycle;
each of R₁₅, R₁₆, and R₁₇ is independently selected from hydrogen and halogen; and wherein at least one of R₁₅, R₁₆, and R₁₇ is a halogen;
bb) a solvent; and
cc) an acid; and
iia) reacting the mixture;
BB) a compound of Formula IV, wherein 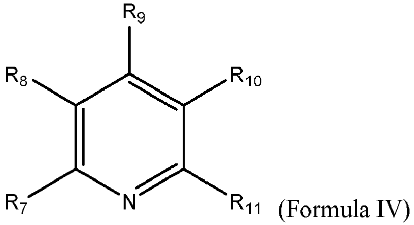 each of R₇ - R₁₁ is independently selected from hydrogen and halogen;
CC) a solvent;
DD) an inorganic base; and
EE) optionally an additive; and
IIA) reacting the mixture;
b) a solvent;
c) a base comprising
a compound comprising a metal; and
optionally a first amine base;
d) optionally an additive; and
e) optionally a second amine base; and
ii) reacting the mixture with an organic compound; and
B) a metal hydroxide; and
II) reacting the mixture.

2. The method of claim 1, wherein:
(A) the metal hydroxide is selected from alkali hydroxide, alkaline earth metal hydroxide, and combinations thereof, or
(B) the method step II) of reacting the mixture occurs at a reaction temperature in the range of about 0 °C to about 90 °C.

3. The method of claim 1, wherein the first amine base and the second amine base are each independently selected from *i*Pr₂NH, TMP, hexamethyldisilazane, Et₂NH, c-hexyl₂NH, and combinations thereof.

4. The method of claim 1, wherein the solvent b) is selected from THF, toluene, 1,4-dioxane, Me-THF, and combinations thereof.

5. The method of claim 1, wherein the method step ii) of reacting the mixture occurs at a reaction temperature in the range of about 0 °C to about 50 °C.

6. The method of claim 1, wherein the compound of Formula VIII is prepared according to a method comprising
I) forming a mixture comprising
A) a compound of Formula VII, wherein 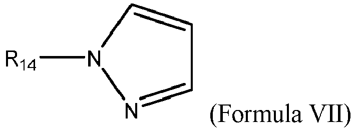 R₁₄ is selected from substituted or unsubstituted carbocycle and substituted or unsubstituted heterocycle;
B) a solvent;
C) a base comprising
a compound comprising a metal; and
optionally a first amine base; and
D) optionally a second amine base; and
II) reacting the mixture with a halogenation reagent.

7. The method of claim 6, wherein the method produces a compound selected from 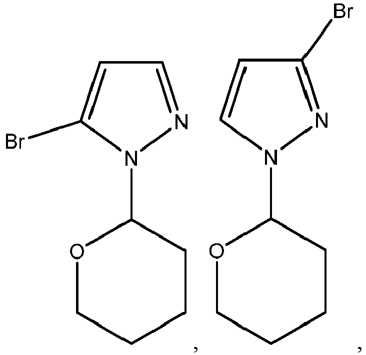 and combinations thereof.

8. A method of preparing a compound of Formula VI, wherein 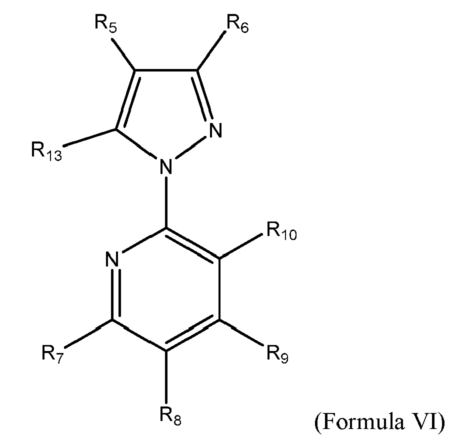
each of R₅ - R₁₀ is independently selected from hydrogen and halogen; and
R₁₃ is an organic acid, the method comprising
I) forming a mixture comprising
A) a compound of Formula III, wherein 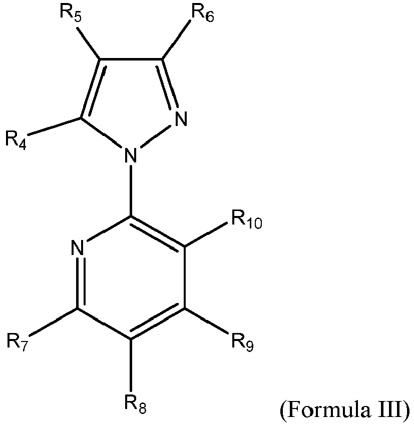
R₄ is hydrogen;
each of R₅ - R₁₀ is independently selected from hydrogen and halogen; and
wherein the compound of Formula III is prepared according to a method comprising
i) forming a mixture comprising
a) a compound of Formula II, wherein 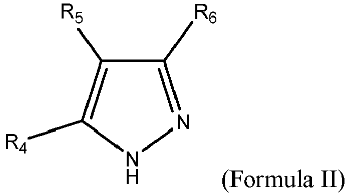
each of R₄, R₅, and R₆ is independently selected from hydrogen and halogen;
wherein at least one of R₄, R₅, and R₆ is hydrogen; and
wherein the compound of Formula II is prepared according to a method comprising
IA) forming a mixture comprising
AA) a compound of Formula VIII, wherein 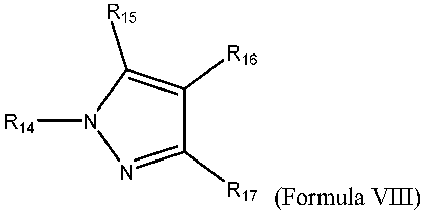
R₁₄ is selected from substituted or unsubstituted carbocycle and substituted or unsubstituted heterocycle;
each of R₁₅, R₁₆, and R₁₇ is independently selected from hydrogen and halogen; and
wherein at least one of R₁₅, R₁₆, and R₁₇ is a halogen;
BB) a solvent; and
CC) an acid; and
IIa) reacting the mixture;
b) a compound of Formula IV, wherein 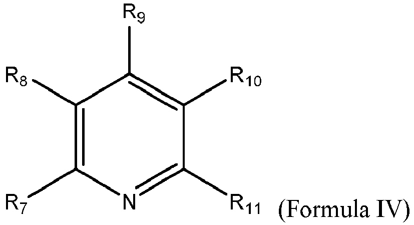 each of R₇ - R₁₁ is independently selected from hydrogen and halogen;
c) a solvent;
d) an inorganic base; and
e) optionally an additive; and
ii) reacting the mixture;
B) a solvent;
C) a base comprising
a compound comprising a metal; and
optionally a first amine base;
D) optionally an additive; and
E) optionally a second amine base; and
II) reacting the mixture with a carbonyl-containing compound.

9. A method of preparing a compound of Formula II, wherein 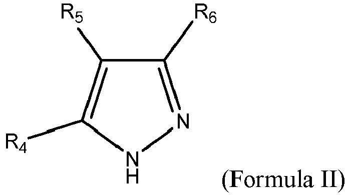
each of R₄, R₅, and R₆ is independently selected from hydrogen and halogen; and
wherein at least one of R₄, R₅, and R₆ is hydrogen, the method comprising
I) forming a mixture comprising
A) a compound of Formula VIII, wherein 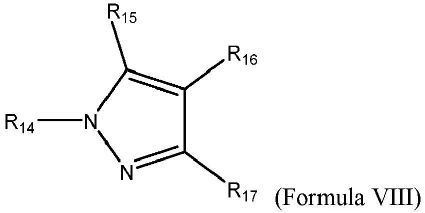
R₁₄ is selected from substituted or unsubstituted carbocycle and substituted or unsubstituted heterocycle;
each of R₁₅, R₁₆, and R₁₇ is independently selected from hydrogen and halogen; and
wherein at least one of R₁₅, R₁₆, and R₁₇ is a halogen;
B) a solvent; and
C) an acid; and
II) reacting the mixture.

10. The method of claim 9, wherein the solvent is selected from methanol, ethanol, isopropanol, toluene, 1,4-dioxane, tetrahydrofuran, and combinations thereof.

11. A method of preparing a compound of Formula VII, wherein 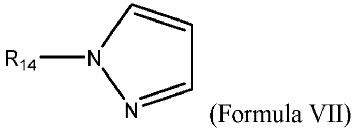 R₁₄ is selected from substituted or unsubstituted carbocycle and substituted or unsubstituted heterocycle, the method comprising
I) forming a mixture comprising
A) pyrazole or a pyrazole derivative;
B) a cycloalkene or heterocycloalkene;
C) optionally a solvent; and
D) an acid; and
II) reacting the mixture.

12. A method of preparing a compound of Formula VIII, wherein 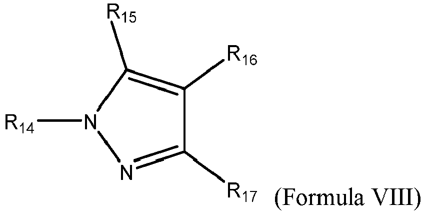
R₁₄ is selected from substituted or unsubstituted carbocycle and substituted or unsubstituted heterocycle;
each of R₁₅, R₁₆, and R₁₇ is independently selected from hydrogen and halogen; and
wherein at least one of R₁₅, R₁₆, and R₁₇ is a halogen, the method comprising
I) forming a mixture comprising
A) the compound of Formula VII, wherein 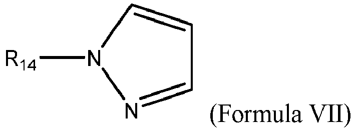 R₁₄ is selected from substituted or unsubstituted carbocycle and substituted or unsubstituted heterocycle;
B) a solvent;
C) a base comprising
a compound comprising a metal; and
optionally a first amine base; and
D) optionally a second amine base; and
II) reacting the mixture with a halogenation reagent.

13. A method of preparing a compound of Formula V, wherein 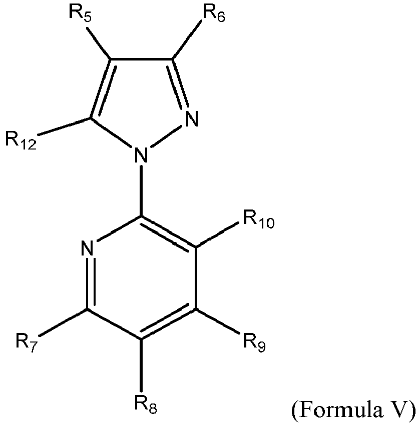
each of R₅ - R₁₀ is independently selected from hydrogen and halogen; and
R₁₂ is selected from ether, ester, and nitrile, the method comprising
i) forming a mixture comprising
a) a compound of Formula III, wherein 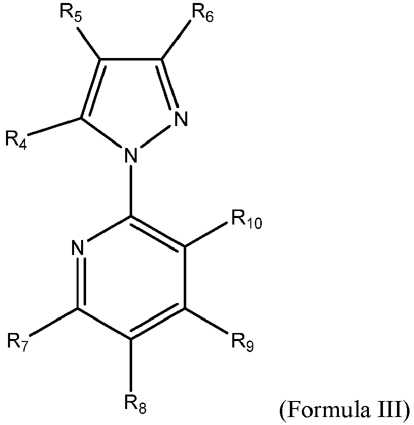
R₄ is hydrogen;
each of R₅ - R₁₀ is independently selected from hydrogen and halogen; and
wherein the compound of Formula III is prepared according to a method comprising
IA) forming a mixture comprising
AA) a compound of Formula II, wherein 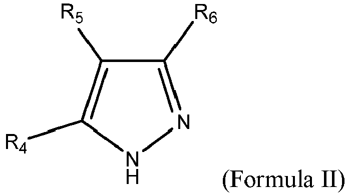
each of R₄, R₅, and R₆ is independently selected from hydrogen and halogen;
wherein at least one of R₄, R₅, and R₆ is hydrogen; and
wherein the compound of Formula II is prepared according to a method comprising
ia) forming a mixture comprising
aa) a compound of Formula VIII, wherein 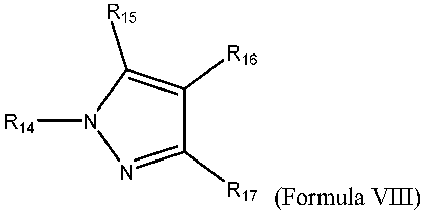
R₁₄ is selected from substituted or unsubstituted carbocycle and substituted or unsubstituted heterocycle;
each of R₁₅, R₁₆, and R₁₇ is independently selected from hydrogen and halogen; and
wherein at least one of R₁₅, R₁₆, and R₁₇ is a halogen;
bb) a solvent; and
cc) an acid; and
iia) reacting the mixture;
BB) a compound of Formula IV, wherein 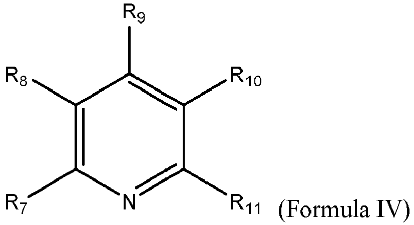 each of R₇ - R₁₁ is independently selected from hydrogen and halogen;
CC) a solvent;
DD) an inorganic base; and
EE) optionally an additive; and
IIA) reacting the mixture;
b) a solvent;
c) a base comprising
a compound comprising a metal; and
optionally a first amine base;
d) optionally an additive; and
e) optionally a second amine base; and
ii) reacting the mixture with an organic compound.

14. The method of any of claims 1, 8, 10 or 12, wherein the compound comprising a metal is a Grignard reagent.

15. A method of preparing a compound of Formula III, wherein 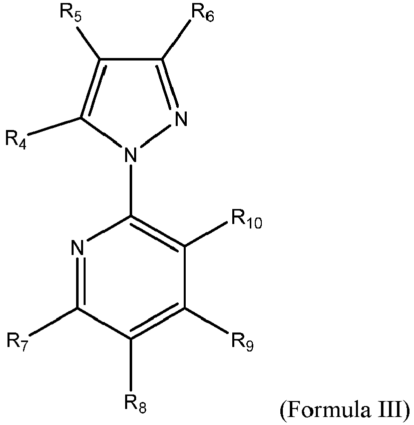
R₄ is hydrogen; and
each of R₅ - R₁₀ is independently selected from hydrogen and halogen, the method comprising
i) forming a mixture comprising
a) a compound of Formula II, wherein 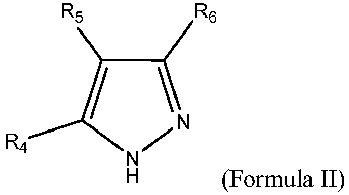
each of R₄, R₅, and R₆ is independently selected from hydrogen and halogen;
wherein at least one of R₄, R₅, and R₆ is hydrogen; and
wherein the compound of Formula II is prepared according to a method comprising
IA) forming a mixture comprising
AA) a compound of Formula VIII, wherein 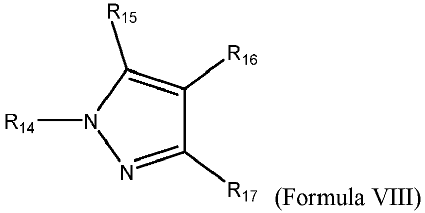
R₁₄ is selected from substituted or unsubstituted carbocycle and substituted or unsubstituted heterocycle;
each of R₁₅, R₁₆, and R₁₇ is independently selected from hydrogen and halogen; and
wherein at least one of R₁₅, R₁₆, and R₁₇ is a halogen;
BB) a solvent; and
CC) an acid; and
IIa) reacting the mixture;
b) a compound of Formula IV, wherein 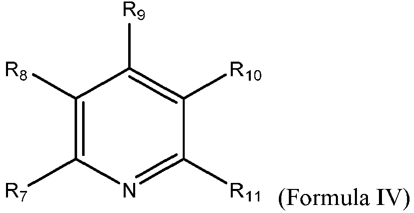 each of R₇ - R₁₁ is independently selected from hydrogen and halogen;
c) a solvent;
d) an inorganic base; and
e) optionally an additive; and
ii) reacting the mixture.
